(19) **Europäisches Patentamt**
**European Patent Office**
**Office européen des brevets**

(11) **EP 4 389 905 A1**

(12) **EUROPEAN PATENT APPLICATION**

(43) Date of publication:
**26.06.2024 Bulletin 2024/26**

(21) Application number: **22215578.0**

(22) Date of filing: **21.12.2022**

(51) International Patent Classification (IPC):
**C12P 7/6463** *(2022.01)* **C13K 1/02** *(2006.01)*
**C13K 13/00** *(2006.01)*

(52) Cooperative Patent Classification (CPC):
**C12P 7/6463;** C12P 2203/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Technische Universität München
80333 München (DE)**

(72) Inventors:
• **Rerop, Zora Selina
85748 Garching (DE)**

• **Stellner, Nikolaus Isidor
80638 Munich (DE)**
• **Masri, Mahmoud
85221 Dachau (DE)**
• **Brück, Thomas
85452 Eichenried (DE)**

(74) Representative: **Engelhard, Markus
Boehmert & Boehmert
Anwaltspartnerschaft mbB
Pettenkoferstrasse 22
80336 München (DE)**

(54) **PRETREATMENT OF SALT-CONTAINING HYDROLYSATE, PARTICULARLY FOR USE IN FERMENTATION PROCESSES**

(57) The present invention relates to a method of reducing a salt content of a hydrolysate which comprises salt. The present invention further relates to a method of producing a target product, preferably a microbial oil, comprising providing a hydrolysate with reduced salt content and cultivating a microorganism, preferably an oleaginous microorganism, using a growth medium comprising the hydrolysate with reduced salt content.

**Figure 2**

EP 4 389 905 A1

**Description**

FIELD OF THE INVENTION

The present invention relates to a method of reducing a salt content of a hydrolysate which comprises salt. The present invention further relates to a method of producing a target product, preferably a microbial oil, comprising providing a hydrolysate with reduced salt content and cultivating a microorganism, preferably an oleaginous microorganism, using a growth medium comprising the hydrolysate with reduced salt content.

BACKGROUND OF THE INVENTION

Single cell oils (SCOs), which are produced by oleaginous microorganisms, can be used as an alternative resource to edible plant oils for the production of advanced biofuels. As opposed to growing crops for oil production, the cultivation of microorganisms is seasonally independent and biotechnological plants allow for land-efficient use and vertical scale up. However, the major weak point of an ecological SCO production is the choice of a cost-effective feedstock. In this context, using industrial waste streams has a high potential to solve the disposal and the feedstock issues of advanced bioprocesses. However, most of industrial waste streams have disadvantages such as comprising compounds which disturb microbial growth which prevents an efficient production of target products such as microbial oils.

The pulp and paper industry is one of the major producers of waste streams with high concentrations of biodegradable carbon. One of the main processes of cellulose fiber production, besides alkaline Kraft-pulping, is acidic sulfite pulping combined with steam explosion to hydrolyze the cells and lignocellulose compounds and to separate the valuable cellulose fibers. The resulting waste stream typically contains high amounts of pentose sugars, smaller amounts of hexose sugars, and uronic acids in addition to aliphatic carboxylic acids, furans, and phenolic compounds. In most industrial plants, it is used for energy production by anaerobic fermentation to methane or direct combustion. Therefore, the value creation of this waste stream is currently limited.

An advanced example of lignocellulosic waste usage for the production of alternative oleochemicals is the production of ethanol with bacteria or yeasts as whole cell biocatalysts. Furthermore, the microbial production of longer chain fatty acids from primary C13 to C21, has been described for microalgae, bacteria, and oleaginous yeast. However, operating oleaginous yeast fermentations in diauxic fermentation modes based on limiting nitrogen, or phosphate concentrations results in reduced growth rates, biomass accumulation and lipid yields. An example of lipid production on lignocellulosic hydrolysate (LCH) with the fermentation host *Rhodosporidium toruloides* reached 39.5 g/l lipid titer in a fed-batch fermentation. However, the given example utilizes hexose sugars as carbon source, as opposed to pentose sugars, that can only be efficiently utilized by C. *oleaginosus*. Furthermore, lignocellulosic hydrolysate typically contains compounds such as salts which inhibit microbial growth and reduce the yield and efficiency.

Side, waste, or and residual stream hydrolysates from lignocellulosic biomass contain a high salt concentration and a high content of compounds inhibiting microbial growth such as soluble oligo and monomeric lignin, furans, and heavy metals which hinder any fermentative process using such side, waste, and/or residual stream. To the current time, combusting after intensive concentration is the only possible and applied utilization of such side, waste or and residual stream hydrolysates.

Thus, there is a need to turn waste streams such as lignocellulosic hydrolysates into suitable growth substrates. Furthermore, there remains the need to provide methods which reduce the salt content of hydrolysates to be used as a growth medium or in a growth medium for a cultivation of microorganisms. There is also a need for converting hydrolysates such as lignocellulosic hydrolysates, e.g. lignocellulosic hydrolysates from acidic pulping, into suitable growth media or growth media supplements. There also remains the need for an efficient method to produce target products such as microbial lipids. Moreover, there is a need to provide an environmentally friendly and cost-efficient method for producing target products such as SCOs.

SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of reducing a salt content of a hydrolysate which comprises salt, preferably a biogenic hydrolysate which comprises salt, comprising:

> a) providing a hydrolysate which comprises salt, preferably a biogenic hydrolysate which comprises salt, more preferably a lignocellulosic hydrolysate which comprises salt;
> b) optionally, neutralizing a pH of the hydrolysate of step a) to obtain a neutralized hydrolysate; wherein, optionally, the neutralized hydrolysate has a pH in a range of from about pH 4 to about pH 8;
> c) adding $CaCO_3$, $Ca(OH)_2$, CaO, MgO, $MgCO_3$, and/or $Mg(OH)_2$, preferably $CaCO_3$ and/or $Ca(OH)_2$, to the hydrolysate of step a) or to the neutralized hydrolysate of step b) to obtain a hydrolysate which comprises precipitated salt; wherein, optionally, the hydrolysate which comprises precipitated salt has a pH in a range of from about pH 4 to about pH 8.5;
> d) adding a chelating agent to the hydrolysate of step c);
> e) optionally, adjusting a pH of the hydrolysate of step c) and/or step d) to a pH in a range of from about pH 3 to about pH 8.5, preferably of from about pH 3.5 to about pH 7.5, more preferably of from about pH 5 to about pH 7, even more preferably of from about pH 6 to about pH 7;
> f) obtaining a hydrolysate with reduced salt content.

In one embodiment, the hydrolysate provided in step a) is a hydrolysate derived from a paper production such as a hydrolysate derived from a pulp production, a hydrolysate derived from forestry, an agricultural hydrolysate, a food hydrolysate, a food waste hydrolysate, a biofuel waste hydrolysate, a textile hydrolysate, an animal tissue hydrolysate, a plant tissue hydrolysate, a microbial biomass hydrolysate, an industrial waste hydrolysate, a municipal waste hydrolysate, or any combination thereof;
wherein, preferably, the hydrolysate is a lignocellulosic hydrolysate, preferably a spent liquor hydrolysate, and/or is a hydrolysate derived from pulping, more preferably is a hydrolysate derived from acidic pulping.
In one embodiment, the hydrolysate provided in step a) is a hydrolysate obtained by a physical treatment, a chemical treatment, an enzymatical treatment, and/or a biological treatment of a substrate, preferably of a biomass;

> wherein, preferably,

> the physical treatment is selected from mechanical treatments, pressure treatments, heat treatments, steam explosions, combustion, and any combination thereof;

> the chemical treatment is selected from an alkaline treatment, an acidic treatment, and a treatment at neutral pH; wherein, preferably, the chemical treatment is a treatment with any of a salt, an acid, a peroxide, and any combination thereof, preferably with a sulfide, a sulfite, and/or a bisulfite;

> the enzymatical treatment is a treatment with one or more enzymes selected from hydrolases, preferably endo- and exo-glycoside hydrolases, glycosylases, peptidases, such as endo- and exo-peptidases, proteases, amylases, dehydrogenases, peroxidases, ligninolytic enzymes, and any combination thereof; and

> the biological treatment is a treatment with a microorganism, preferably a treatment with a microorganism selected from bacteria, yeast, and fungi.

In one embodiment, the hydrolysate provided in step a) comprises salt in an amount in a range of from about 0.0001 mol/l to about 15 mol/l, preferably of from about 0.0005 mol/l to about 8 mol/l, and/or carbon in an amount in a range of from about 0.1% by weight to about 65% by weight.
In one embodiment, the hydrolysate provided in step a) comprises a salt selected from a sulfate, a sulfide, a sulfite, a nitrate, a nitrite, a chloride, and any combination thereof; wherein, preferably, the salt comprises a sulfate, a sulfide, and/or a sulfite.
In one embodiment, the hydrolysate provided in step a) comprises a lignol, a lignan, an organic acid, and/or a sugar; wherein, optionally, said sugar comprises xylose, glucose, mannose, and/or galactose; wherein, preferably, said sugar comprises monosaccharides, preferably xylose; and wherein, preferably, said organic acid comprises acetic acid.
In one embodiment, the chelating agent is selected from $M_3PO_4$, $M_2HPO_4$, $MH_2PO_4$, $MHPO_4$, $MPO_4$, $(NH_4)(H_2PO_4)$, and any combination thereof, wherein M is a metal;

> wherein, preferably, the chelating agent is selected from $Na_3PO_4$, $Na_2HPO_4$, $NaH_2PO_4$, $K_3PO_4$, $K_2HPO_4$, $KH_2PO_4$, $Ca(H_2PO_4)_2$, $CaHPO_4$, $Ca_3(PO_4)_2$, $(NH_4)(H_2PO_4)$, and $Na_3PO_4$;
> wherein, more preferably, the chelating agent is $KH_2PO_4$.

In one embodiment, the method comprises step e) of adjusting a pH of the hydrolysate, and the adjusting is performed by adding NaOH, KOH, CH$_3$COOH, HCl, KCl, sulfuric acid, phosphoric acid, acetic acid, hydrocyanic acid, carbonic acid, or any combination thereof, preferably by adding NaOH and/or KOH, to the hydrolysate of step c) and/or step d).

In one embodiment, the method further comprises a sterilization, preferably of the hydrolysate with reduced salt content obtained in step f), to obtain a sterile hydrolysate with reduced salt content;

wherein, preferably, the sterilization comprises a thermal sterilization, an ultra-high temperature processing, and/or a sterile filtration.

In a further aspect, the present invention relates to a method of producing a target product, preferably a microbial oil, comprising the steps:

i) providing a hydrolysate with reduced salt content by performing a method of reducing a salt content of a hydrolysate which comprises salt, as defined herein;

ii) cultivating a microorganism, preferably an oleaginous microorganism, using a growth medium comprising or consisting of the hydrolysate provided in step i), thereby allowing the microorganism to produce the target product; preferably the oleaginous microorganism to produce microbial oil;

iii) optionally, enzymatically treating the microorganism, preferably the oleaginous microorganism; wherein, optionally, said enzymatically treating comprises performing an enzymatic treatment of said microorganism without any solvent-based extraction or chemicals-based demulsification;

iv) obtaining the target product, preferably microbial oil.

In one embodiment, the microorganism is an oleaginous microorganism, preferably an oleaginous yeast, more preferably a *Cutaneotrichosporon sp.,* even more preferably *Cutaneotrichosporon oleaginosus.*

In one embodiment, the target product is selected from microbial oils, glycerol, free fatty acids, mono- di- and triglycerides, phospholipids, sphingolipids, polyols, alcohols, organic acids, biodiesel, hydrogen, methane, biopolymers, carotenoids, cellulose, squalene, sterols, vitamins, phenolic compounds, pigments, peptides, proteins such as enzymes, DNA, RNA, and any combination thereof;

wherein, preferably, the target product comprises microbial oil.

In one embodiment, the hydrolysate with reduced salt content provided in step i) comprises acetic acid and/or xylose,

wherein, preferably, the hydrolysate comprises or consists of a lignocellulosic hydrolysate.

In one embodiment, the cultivating in step ii) comprises adding acetic acid and/or a carbon source other than acetic acid to the growth medium;

wherein, preferably,

said acetic acid is added in the form of a feed comprising or consisting of acetic acid, wherein, preferably, a concentration of acetic acid in said feed is in a range of from 1 mol/l to 20 mol/l, preferably of from 1.75 mol/l to 15.75 mol/l, wherein, optionally, said feed further comprises a carbon source other than acetic acid.

In one embodiment, the growth medium comprises a sugar such as xylose in an amount in a range of from about 0.1 g/l to about 250 g/l, preferably < 100 g/l; and/or the growth medium comprises acetic acid in an amount in a range of from about 0.01 g/l to about 100 g/l, preferably of from about 1 g/l to about 50 g/l, more preferably of from about 5 g/l to about 10 g/l.

In a further aspect, the present invention relates to a hydrolysate with reduced salt content obtained and/or obtainable by a method of reducing a salt content of a hydrolysate which comprises salt, as defined herein.

In a further aspect, the present invention relates to a composition obtained and/or obtainable by a method of producing a target product, as defined herein.

In one embodiment, said composition comprises or consists of the target product obtained and/or obtainable by a method of producing a target product, as defined herein.

DETAILED DESCRIPTION

The present invention aims at providing hydrolysate with reduced salt content which can be used as a growth medium. Advantageously, the method of reducing a salt content of a hydrolysate allows valorizing products such as biomass and/or waste products such that these products become suitable growth media and/or suitable additives for growth media. Advantageously, waste products, such as spent liquor or other hydrolysates, comprising salt(s) which disturb microbial growth, may be processed into valuable hydrolysates with reduced salt content which may be used as a growth medium or in a growth medium for microbial growth. The method of reducing a salt content of a hydrolysate

EP 4 389 905 A1

effectively upcycles waste products such as biogenic waste.

The inventors have surprisingly found that target products can be efficiently produced with microorganisms cultivated using hydrolysates with reduced salt content, such as lignocellulosic hydrolysates with reduced salt content, as a growth substrate. The inventors have found that, unexpectedly, the method of producing a target product of the invention allows producing target products with high yield. Furthermore, the inventors have found that waste hydrolysates, such as spent liquor, can be valorized as a growth substrate for microorganism cultivation by reducing the salt content. The method of reducing a salt content of a hydrolysate is highly effective in producing hydrolysates which can be used as a growth medium and/or in a growth medium. The present invention allows for the first time the usage of side, waste, and/or residual streams from lignocellulosic biomass for cultivating microorganisms after reducing the salt content.

Interestingly, the inventors found out that the microorganisms demonstrate a unique ability to grow perfectly on the salt-reduced hydrolysate showing a high tolerance and uptake ability to a high content of soluble oligo and monomeric lignin, and furans. With the current fermentation system the microorganisms demonstrate a much higher growth rate at real salt-reduced lignocellulosic hydrolysate in comparison to the model lignocellulosic hydrolysate.

The present invention relates to a method of reducing a salt content of a hydrolysate which comprises salt, preferably a biogenic hydrolysate which comprises salt, comprising:

a) providing a hydrolysate which comprises salt, preferably a biogenic hydrolysate which comprises salt, more preferably a lignocellulosic hydrolysate which comprises salt;
b) optionally, neutralizing a pH of the hydrolysate of step a) to obtain a neutralized hydrolysate; wherein, optionally, the neutralized hydrolysate has a pH in a range of from about pH 4 to about pH 8;
c) adding $CaCO_3$, $Ca(OH)_2$, $CaO$, $MgO$, $MgCO_3$, and/or $Mg(OH)_2$, preferably $CaCO_3$ and/or $Ca(OH)_2$, to the hydrolysate of step a) or to the neutralized hydrolysate of step b) to obtain a hydrolysate which comprises precipitated salt; wherein, optionally, the hydrolysate which comprises precipitated salt has a pH in a range of from about pH 4 to about pH 8.5;
d) adding a chelating agent to the hydrolysate of step c);
e) optionally, adjusting a pH of the hydrolysate of step c) and/or step d) to a pH in a range of from about pH 3 to about pH 8.5, preferably of from about pH 3.5 to about pH 7.5, more preferably of from about pH 5 to about pH 7, even more preferably of from about pH 6 to about pH 7;
f) obtaining a hydrolysate with reduced salt content.

In one embodiment, the method reduces the salt content, e.g. sulfate content, of the hydrolysate which comprises salt by at least 1%, preferably by at least 10%, more preferably by at least 30%, even more preferably by at least 50%, even more preferably by at least 80%. In one embodiment, said reducing a salt content comprises a significant reduction of the salt content, preferably by at least 1%, preferably by at least 10%, more preferably by at least 30%, even more preferably by at least 50%, even more preferably by at least 80%.

In one embodiment, the term "reducing a salt content", as used herein, relates to reducing a total salt content of a hydrolysate and/or reducing the amount of a particular salt, particularly reducing the amount of at least one salt, in said hydrolysate, e.g. relates to reducing the sulfate content of a hydrolysate. In one embodiment, the method of reducing a salt content comprises reducing a sulfate content, and optionally further comprises reducing a calcium content of said hydrolysate. In one embodiment, the salt(s) composition of said hydrolysate is changed independent of the total salt content. For example, the amount of sulfate present in said hydrolysate may be reduced by the method of the invention (thus changing the total salt(s) composition of the hydrolysate), while the total salt content of the hydrolysate is maintained or is changed. In one embodiment, the terms "content", "amount", and "concentration" are used interchangeably. In one embodiment, the method of reducing a salt content of a hydrolysate comprises reducing a salt content of at least one salt in said composition, e.g. reducing a sulfate content. In one embodiment, the method of reducing a salt content of a hydrolysate comprises reducing the salt concentration, e.g. sulfate concentration, of at least one salt present in said hydrolysate; wherein, optionally, the total salt content of said hydrolysate is reduced. In one embodiment, the salt reduced in a method of reducing a salt content of a hydrolysate is sulfate. In one embodiment, reducing a salt content comprises or consists of reducing a sulfate content. In one embodiment, the method of reducing a salt content of a hydrolysate which comprises salt is a method of reducing a sulfate content of a hydrolysate which comprises sulfate. In one embodiment, the method of reducing a salt content of a hydrolysate which comprises salt further comprises reducing a calcium concentration e.g. of said hydrolysate. In one embodiment, the hydrolysate with reduced salt content obtained in step f) of a method of reducing a salt content is a hydrolysate with reduced sulfate content, optionally with reduced calcium content. In one embodiment, the hydrolysate with reduced salt content obtained in step f) of a method of reducing a salt content has a salt composition which differs from the salt composition of the hydrolysate provided in step a); wherein, preferably, the concentration of at least one salt, e.g. a sulfate concentration, is reduced in said hydrolysate obtained in step f) compared to the concentration of said at least one salt in the hydrolysate provided in step a); wherein, optionally, the total salt content of said hydrolysate obtained in step f) is the same or is different from the total salt content of the hydrolysate provided in step a).

The term "hydrolysate", as used herein in the context of a method for reducing a salt content of a hydrolysate according to the invention, relates to any product of hydrolysis, particularly to a product of a hydrolysis of a biomass, such as of a lignocellulosic biomass. For example, the hydrolysate may be derived from solid paper waste, pulp, spent liquor, wood, sawdust, plants such as crops, straw, food, food waste, biofuel waste, textiles, animal tissue, microbial biomass, municipal waste, and/or industrial waste. In one embodiment, the hydrolysate comprises sulfate. Hydrolysates may comprise carbohydrates, particularly sugars, sugar degradation products, and/or lignin degradation products. In one embodiment, the hydrolysate comprises sugar, particularly xylose, and/or acetic acid. In one embodiment, the hydrolysate is derived from and/or prepared from lignocellulosic biomass. In one embodiment, the terms "derive from" and "prepare from", or "derived from" and "prepared from", are used interchangeably. In one embodiment, the term "derived from", as used herein, in the context of a hydrolysate, refers to the hydrolysate being or having been prepared, particularly obtained, from a starting material, such as a biomass, by any method known to the skilled person, preferably by a physical treatment, a chemical treatment, an enzymatical treatment, and/or a biological treatment of a starting material, particularly of a substrate, preferably of a biomass. In one embodiment, the starting material, particularly the substrate, comprises or consists of a product of a paper production, such as pulp, spent liquor, bleaching waste, paper, and/or paper waste; a forestry product, such as wood and/or sawdust; an agricultural product, such as straw; a food product or a food waste, such as bread; a biofuel production waste, such as microbial biomass; a hydrogen production waste, such as microbial biomass; a textile, optionally a biodegradable textile, such as wool, cotton, and/or hemp; an animal tissue, such as meat; a plant biomass, such as a crop; a microbial biomass, such as fungal biomass, bacterial biomass, and/or yeast biomass; an industrial waste, optionally a biodegradable industrial waste, such as a hemp hydrolysate; a municipal waste; or any combination thereof. In one embodiment, the starting material, particularly the substrate, comprises or consists of a lignocellulosic biomass. In one embodiment, a biofuel waste comprises or consists of a hydrogen production waste. For example, biofuel production may relate to hydrogen production. In one embodiment, a biofuel waste hydrolysate is a hydrogen production waste hydrolysate.

For example, biomass may relate to a biodegradable fraction of a product, waste, and/or residue of biological origin, e.g. derived from agriculture, forestry, and/or related industries, including fisheries and aquaculture. For example, biomass may relate to a biodegradable part of waste from industry and households. In one embodiment, said industrial waste is selected from the group consisting of side stream and/or waste stream from food processing, pulp production, paper production, agro-industry, biofuel, and/or forestry. In one embodiment, said microbial biomass is selected from bacterial biomass, fungal biomass, yeast biomass, microalgae biomass, and combinations thereof; wherein, preferably, said microbial biomass is fungal biomass, particularly yeast biomass. In one embodiment, said fungal biomass comprises or consists of biomass of a microorganism selected from the group consisting of *Aspergillus sp., Fusarium sp., Trichoderma sp., Ascobolus sp., Rhizopus sp.,* and combinations thereof. For example, said fungal biomass may comprise *Trichoderma reesei* and /or *Aspergillus niger* biomass. In one embodiment, said yeast biomass comprises or consists of biomass of a microorganism selected from the group consisting of *Saccharomyces sp., Yarrowia sp., Rhodosporidium sp., Cryptococcus sp., Trichosporon sp., Lipomyces sp., Rhodotorula sp., Candida sp., Cutaneotrichosporon sp.,* and combinations of any of the foregoing; wherein, preferably, said yeast biomass comprises *Cutaneotrichosporon oleaginosus* and/or *Saccharomyces cerevisiae* biomass.

The term "lignocellulosic biomass" is meant to refer to a biomass comprising cellulose, hemicellulose, lignin, and combinations thereof. In one embodiment, the hydrolysate is derived from a product of a paper production, such as pulp, spent liquor, paper, and/or paper waste; a forestry product, such as wood and/or sawdust; an agricultural product, such as straw; a food product or a food waste, such as bread; a biofuel production waste, such as microbial biomass; a hydrogen production waste, such as microbial biomass; a textile, optionally a biodegradable textile, such as wool, cotton, and/or hemp; an animal tissue, such as meat; a plant biomass, such as a crop; a microbial biomass, such as fungal biomass, bacterial biomass, and/or yeast biomass; an industrial waste, optionally a biodegradable industrial waste, such as a hemp hydrolysate; a municipal waste; or any combination thereof.

In one embodiment, the hydrolysate is a hydrolysate derived from a paper production such as a pulp or pulp hydrolysate, spent liquor, a paper hydrolysate, and/or paper waste hydrolysate; a hydrolysate derived from forestry, such as a wood hydrolysate and/or sawdust hydrolysate; an agricultural hydrolysate, such as a straw hydrolysate; a food hydrolysate, such as a fruit peel hydrolysate; a food waste hydrolysate, such as a bread residue hydrolysate; a biofuel production waste hydrolysate, such as a microbial biomass hydrolysate; a hydrogen production waste, such as a microbial biomass hydrolysate; a textile hydrolysate, such as a wool hydrolysate, a cotton hydrolysate, and/or a hemp hydrolysate; an animal tissue hydrolysate, such as a meat hydrolysate; a plant tissue hydrolysate, such as a crop hydrolysate; a microbial biomass hydrolysate, such as a fungal biomass hydrolysate, a bacterial biomass hydrolysate, and/or a yeast biomass hydrolysate; an industrial waste hydrolysate, such as a hemp hydrolysate; a municipal waste hydrolysate; or any combination thereof.

The term "spent liquor", as used herein, relates to any spent liquor known to the person skilled in the art, and particularly relates to a liquid effluent from the digestion of wood during pulping. Spent liquor typically comprises wood components, such as lignin, and further comprises a digestant, such as caustic, sulfite, or sulfate. The term "pulp", as used herein, refers to pulp of a paper production process, particularly to a lignocellulosic fibrous material prepared by chemically or mechanically separating cellulose fibers from raw materials such as wood, fiber crops, waste paper, or rags. Pulp is a major raw material used in papermaking and the industrial production of other paper products. Pulp production may comprise mechanical pulping, thermomechanical pulping, chemi-thermomechanical pulping, chemical pulping, organosolv pulping. Chemical pulping may comprise a Kraft process, a sulfite process, and/or a soda pulping process. In one embodiment, a hydrolysate derived from a pulp production is a hydrolysate derived from chemical pulping, preferably acidic pulping. In one embodiment, the hydrolysate is or is derived from a spent liquor.

In a preferred embodiment, the hydrolysate is derived from biomass, such as lignocellulosic biomass. In one embodiment, the hydrolysate is a hydrolysate derived from a paper production such as a hydrolysate derived from a pulp production, a hydrolysate derived from forestry, an agricultural hydrolysate, a food hydrolysate, a food waste hydrolysate, a biofuel waste hydrolysate, a textile hydrolysate, an animal tissue hydrolysate, a plant tissue hydrolysate, a microbial biomass hydrolysate, an industrial waste hydrolysate, a municipal waste hydrolysate, or any combination thereof. In one embodiment, the hydrolysate is a biogenic hydrolysate. In one embodiment, the term "biogenic hydrolysate", as used herein, relates to a hydrolysate of a biogenic product such as biogenic waste. A biogenic product is a product made by or of life forms. In one embodiment, the biogenic hydrolysate comprises or consists of a lignocellulosic hydrolysate. In a preferred embodiment, the hydrolysate is or is derived from acidic pulping, particularly is or is derived from a waste stream and/or residual stream from acidic pulping. Advantageously, by the method of the invention, the pentose sugars in waste streams and/or residual streams from acidic pulping become valorized as a feedstock for cultivating microorganisms such as *Cutaneotrichosporon sp.* Furthermore, the salt content of growth-inhibiting salts, such as sulfates, is advantageously reduced by a method of the invention. By reducing the salt content of a hydrolysate, e.g. reducing the sulfate content of the hydrolysate, the hydrolysate becomes useful as a feedstock, e.g. for cultivating microorganisms.

In one embodiment, the hydrolysate with reduced salt content provided in step i) comprises volatile organic acids, xylose, and/or glucose; wherein, preferably, the hydrolysate comprises or consists of a lignocellulosic hydrolysate. For example, said volatile organic acids may comprise acetic acid. In one embodiment, the cultivating in step ii) comprises adding volatile organic acid e.g. acetic acid, a carbon source other than volatile organic acid, and/or additional nutrients to the growth medium; wherein, preferably, said volatile organic acid is added in the form of a feed comprising or consisting of said volatile organic acid; wherein, preferably, a concentration of volatile organic acid in said feed is in a range of from 1 mol/l to 20 mol/l, preferably of from 1.75 mol/l to 15.75 mol/l; wherein, optionally, said feed further comprises a carbon source other than said volatile organic acid, such as a carbon source other than acetic acid.

In one embodiment, the hydrolysate is or is derived from a waste stream of a paper production, preferably is or is derived from a pentose sugar fraction of a waste stream of a paper production. In one embodiment, the hydrolysate which comprises salt, e.g. a pentose sugar fraction of a waste stream of a paper production, comprises:

- hexose sugars in an amount of from 0.5 % to 5 %, pentose sugars in an amount of from 1% to 10%, oligosaccharides in an amount of from 0.3 % to 1 %, volatile fatty acids in an amount of from 0.1% to 3%, and soluble lignin in an amount of from 0.2 % to 1%; or
- hexose sugars in an amount of from 5% to 25%, pentose sugars in an amount of from 30% to 65%, oligosaccharides in an amount of from 2% to 15%, %, volatile fatty acids in an amount of from 0.5% to 25%, and soluble lignin in an amount of from 1% to 10%; or
- hexose sugars in an amount of from 15% to 35%, pentose sugars in an amount of from 1% to 25%, oligosaccharides in an amount of from 10% to 40%, volatile fatty acids in an amount of from 3% to 25%, and soluble lignin in an amount of from 5% to 25%.

In one embodiment, the hydrolysate is a hydrolysate obtained by a physical treatment, a chemical treatment, an enzymatical treatment, and/or a biological treatment of a substrate, preferably of a biomass; wherein, optionally, the substrate is selected from a product of a paper production, such as pulp, spent liquor, paper, and/or paper waste, a forestry product, such as wood and/or sawdust, an agricultural product, such as straw, a food product or a food waste, such as bread, a biofuel production waste, such as microbial biomass, a hydrogen production waste, such as microbial biomass, a textile, such as wool, cotton, and/or hemp, an animal tissue, such as meat, a plant biomass, such as a crop, a microbial biomass, such as fungal biomass, bacterial biomass, and/or yeast biomass, an industrial waste, such as a hemp hydrolysate, a municipal waste, and any combination thereof. In one embodiment, the hydrolysate is provided by performing a physical treatment, a chemical treatment, an enzymatical treatment, and/or a biological treatment of a substrate, preferably of a biomass. In one embodiment, the substrate is selected from a product of a paper production, such as pulp, spent liquor, paper, and/or paper waste, a forestry product, such as wood and/or sawdust, an agricultural product, such as straw, a food product or a food waste, such as bread, a biofuel production waste, such as microbial biomass, a hydrogen production waste, such as microbial biomass, a textile, such as wool, cotton, and/or hemp, an animal tissue, such as meat, a plant biomass, such as a crop, a microbial biomass, such as fungal biomass, bacterial biomass, and/or yeast biomass, an industrial waste, such as a hemp hydrolysate, a municipal waste, and any combination thereof. In one embodiment, the substrate is a lignocellulosic biomass. In one embodiment, the hydrolysate is a lignocellulosic hydrolysate derived from wood, preferably from hardwood.

In one embodiment, the physical treatment is selected from mechanical treatments, pressure treatments, heat treatments, steam explosions, combustion, and any combination thereof. In one embodiment, the chemical treatment is selected from an alkaline treatment, an acidic treatment, and a treatment at neutral pH; wherein, preferably, the chemical treatment is a treatment with any of a salt, an acid, a peroxide, and any combination thereof, preferably with a sulfide, a sulfite, and/or a bisulfite. In one embodiment, the enzymatical treatment is a treatment with one or more enzymes selected from hydrolases, preferably endo- and exo-glycoside hydrolases, glycosylases, peptidases, such as endo- and exo-peptidases, proteases, amylases, dehydrogenases, peroxidases, ligninolytic enzymes, and any combination thereof. In one embodiment, the biological treatment is a treatment with a microorganism, preferably a treatment with a microorganism selected from bacteria, yeast, and fungi. For example, the hydrolysate may be provided by a chemical and a physical treatment, e.g. an acidic or alkaline treatment followed by steam explosion. For example, the enzymatical treatment may be performed with any of LiP (EC 1.11.1.14), MnP (EC 1.11.1.13), laccase (EC 1.10.3.2), b-O-4 ether cleaving enzymes such as b-etherase, b-O-4 aryl-ether cleaving enzymes, O-demethylation enzymes, $H_2O_2$-generating oxidases, aryl-alcohol oxidase (EC 1.1.3.7), quinone reductases (EC 1.6.5.5), cellobiose dehydrogenase (EC 1.1.99.18), catechol 2,3-dioxygenase (EC 1.13.11.2), perhydrolases, lipases (EC 3.1.1.3), and/or any combination thereof.

In one embodiment, the hydrolysate which comprises salt comprises salt in an amount in a range of from about 0.0001 mol/l to about 15 mol/l, preferably of from about 0.0005 mol/l to about 8 mol/l, such as a salt selected from a sulfate, a sulfide, a sulfite, a nitrate, a nitrite, a chloride, and any combination thereof. In one embodiment, the hydrolysate which comprises salt comprises a sulfate in an amount in a range of from about 0.0005 mol/l to about 8 mol/l, optionally in a range of from about 0.05 mol/l to about 0.4 mol/l, e.g. of about 0.2 mol/l. In one embodiment, the hydrolysate which comprises salt comprises sulfate and optionally further salts.

In one embodiment, the hydrolysate which comprises salt comprises a salt selected from a sulfate, a sulfide, a sulfite, a nitrate, a nitrite, a chloride, and any combination thereof, preferably comprises sulfate. In a preferred embodiment, the hydrolysate which comprises salt comprises a salt selected from a sulfate, a sulfide, a sulfite, and any combination thereof. The hydrolysate may comprise salt, particularly a high amount of salt such as 8 mol/l, as a result of the preparation of the hydrolysate, such as a chemical treatment e.g. an acidic pulping process.

In one embodiment, the hydrolysate which comprises salt comprises carbon in an amount in a range of from about 0.1% to about 65% by weight, preferably in a range of from about 0.1% to about 35% by weight, e.g. of about 25% by weight; wherein, preferably, "by weight" relates to a dry weight of the hydrolysate. In a preferred embodiment, said carbon is present in said hydrolysate in the form of biodegradable carbon sources, such as sugars and organic acids. In one embodiment, said carbon is present in said hydrolysate in the form of sugars, such as xylose, glucose, mannose, and/or galactose, and/or in the form of organic acids such as acetic acid. In one embodiment, the hydrolysate comprises sugars, such as xylose, glucose, mannose, and/or galactose, and/or comprises acetic acid. In one embodiment, the hydrolysate provided in step a) comprises a lignol, a lignan, an organic acid, and/or a sugar; wherein, optionally, said sugar comprises or consists of xylose, glucose, mannose, and/or galactose; wherein, preferably, said sugar comprises monosaccharides, preferably xylose; and wherein, preferably, said organic acid comprises acetic acid. In one embodiment, the hydrolysate provided in step a) comprises lignols, lignans, organic acids, and/or sugars; wherein, optionally, said sugars comprise or consist of xylose, glucose, mannose, and/or galactose; wherein, preferably, said sugars comprise monosaccharides, preferably xylose; and wherein, preferably, said organic acids comprise acetic acid.

In one embodiment, the hydrolysate comprises sugar in an amount in a range of from about 10 g/l to about 250 g/l, e.g. of about 115 g/l. In one embodiment, the hydrolysate comprises xylose in an amount in a range of from about 30 g/l to about 100 g/l, e.g. of about 77 g/l. In one embodiment, the hydrolysate comprises glucose in an amount in a range of from about 0 g/l to about 20 g/l, such as in a range of from about 0.05 g/l to about 20 g/l, e.g. of about 12 g/l. In one embodiment, the hydrolysate comprises acetic acid in an amount in a range of from about 0 g/l to about 20 g/l, such as in a range of from about 0.05 g/l to about 20 g/l, e.g. of about 12 g/l. In one embodiment, the hydrolysate comprises furans in an amount in a range of from about 0 g/l to about 10 g/l, such as in a range of from about 0.05 g/l to about 10 g/l, e.g. of about 5 g/l. In one embodiment, the hydrolysate comprises lignin-derived compounds in an amount in a range of from about 0 g/l to about 200 g/l, such as in a range of from about 0.05 g/l to about 200 g/l, e.g. of about 90 g/l.

In one embodiment, the terms "hydrolysate", "hydrolysate which comprises salt", and "hydrolysate provided in step a)", are used interchangeably. In one embodiment, the hydrolysate with reduced salt content obtained in step f) differs from the hydrolysate provided in step a) only in the salt content, and optionally in the pH and/or in sterilization. The hydrolysate with reduced salt content obtained in step f) may have any features described with respect to the hydrolysate provided in step a).

In one embodiment, the method of reducing a salt content comprises neutralizing a pH of the hydrolysate of step a) to obtain a neutralized hydrolysate. In one embodiment, the neutralized hydrolysate has a pH in a range of from about pH 4 to about pH 8, preferably in a range of from about pH 6 to about pH 8, more preferably in a range of from about pH 6.5 to about pH 7.5. In one embodiment, the neutralized hydrolysate obtained in step b) has a pH of about pH 4.0, of about pH 4.5, of about pH 5.0, of about pH 5.5, of about pH 6.0, of about pH 6.5, of about pH 7.0, or of about pH 7.5. In one embodiment, said neutralizing a pH of the hydrolysate comprises increasing the pH of the hydrolysate, such as up to pH 7.5 or pH 8. In one embodiment, said neutralizing comprises neutralizing the hydrolysate of step a) by increasing the pH of the hydrolysate to obtain a neutralized hydrolysate, such as to obtain a hydrolysate with a pH of up to pH 7.5 or of up to pH 8. In one embodiment, the neutralized hydrolysate has a pH of about pH 7.

The method of reducing salt content comprises a step of adding $CaCO_3$, $Ca(OH)_2$, $CaO$, $MgO$, $MgCO_3$, and/or $Mg(OH)_2$, preferably $CaCO_3$ and/or $Ca(OH)_2$, to the hydrolysate of step a) or to the neutralized hydrolysate of step b) to obtain a hydrolysate which comprises precipitated salt. The inventors have found that the addition of $CaCO_3$, $Ca(OH)_2$, $CaO$, $MgO$, $MgCO_3$, and/or $Mg(OH)_2$ allows providing a hydrolysate which is suitable for being used as a growth substrate for microorganisms. Particularly, by adding $CaCO_3$, $Ca(OH)_2$, $CaO$, $MgO$, $MgCO_3$, $Mg(OH)_2$, or a combination thereof, growth disturbing substances, particularly salts, are sufficiently removed and a suitability of the hydrolysate for being used as a growth substrate is established. Advantageously, $CaCO_3$, $Ca(OH)_2$, $CaO$, $MgO$, $MgCO_3$, $Mg(OH)_2$, or the combination thereof, serve overliming agents which facilitate the precipitation of the salt present in the hydrolysate. In one embodiment, the hydrolysate which comprises precipitated salt obtained in step c) has a pH in a range of from about pH 4 to about pH 8.5, such as a pH of about pH 4.0, of about pH 4.5, of about pH 5.0, of about pH 5.5, of about pH 6.0, of about pH 6.5, or of about pH 7.0. In one embodiment, said adding $CaCO_3$, $Ca(OH)_2$, $CaO$, $MgO$, $MgCO_3$, $Mg(OH)_2$, or a combination thereof, to the hydrolysate of step a) or to the neutralized hydrolysate of step b) comprises adding said $CaCO_3$, $Ca(OH)_2$, $CaO$, $MgO$, $MgCO_3$, $Mg(OH)_2$, or the combination thereof, in an amount in a range of from 0.001 g/l to 500 g/l, preferably in a range of from 1 g/l to 150 g/l. In one embodiment, said adding comprises adding $CaCO_3$, $Ca(OH)_2$, $CaO$, $MgO$, $MgCO_3$, $Mg(OH)_2$, or the combination thereof, in an amount such that at least 1%, preferably at least 10%, more preferably at least 30%, even more preferably at least 50%, even more preferably at least 80% of the salt content of the hydrolysate of step a) or of the neutralized hydrolysate of step b) precipitates.

In one embodiment, said adding comprises mixing, preferably thoroughly mixing, said $CaCO_3$, $Ca(OH)_2$, $CaO$, $MgO$, $MgCO_3$, and/or $Mg(OH)_2$ with said hydrolysate to facilitate the precipitation of salt(s). In one embodiment, said method comprises mixing said $CaCO_3$, $Ca(OH)_2$, $CaO$, $MgO$, $MgCO_3$, and/or $Mg(OH)_2$ with said hydrolysate, such as by stirring, shaking, and/or vortexing. The inventors have found that by adding $CaCO_3$, $Ca(OH)_2$, $CaO$, $MgO$, $MgCO_3$, $Mg(OH)_2$, or a combination thereof, to a hydrolysate which comprises salt, the salt of the hydrolysate efficiently precipitates, thus providing a hydrolysate which is useful as a growth substrate.

The method of the invention comprises adding a chelating agent to the hydrolysate of step c). The chelating agent may be any chelating agent known to the person skilled in the art, such as chelating agents selected from $M_3PO_4$, $M_2HPO_4$, $MH_2PO_4$, $MHPO_4$, $MPO_4$, $(NH_4)(H_2PO_4)$, EDTA, EGTA, EHPG, and any combination thereof, wherein M is a metal or an alternative counterion.

In one embodiment, the chelating agent is selected from $M_3PO_4$, $M_2HPO_4$, $MH_2PO_4$, $MHPO_4$, $MPO_4$, $(NH_4)(H_2PO_4)$, and any combination thereof, wherein M is a metal. Advantageously, when the chelating agent is selected from $M_3PO_4$, $M_2HPO_4$, $MH_2PO_4$, $MHPO_4$, $MPO_4$, $(NH_4)(H_2PO_4)$, and any combination thereof, the obtained hydrolysate with reduced salt content allows for an efficient growth of microorganisms, such as for the production of target products. The inventors have found that the cultivation of microorganisms, particularly the production of target products, is highly efficient if the hydrolysate with reduced salt content used as a growth substrate is prepared using a chelating agent selected from $M_3PO_4$, $M_2HPO_4$, $MH_2PO_4$, $MHPO_4$, $MPO_4$, $(NH_4)(H_2PO_4)$, and any combination thereof, wherein M is a metal. The inventors have found that the hydrolysate is particularly useful for subsequently being used as a growth substrate, if the chelating agent is selected from said $M_3PO_4$, $M_2HPO_4$, $MH_2PO_4$, $MHPO_4$, $MPO_4$, $(NH_4)(H_2PO_4)$, and any combination thereof, since other chelating agents, such as EDTA, EGTA, and EHPG, may inhibit microbial growth.

In a preferred embodiment, the chelating agent is selected from $Na_3PO_4$, $Na_2HPO_4$, $NaH_2PO_4$, $K_3PO_4$, $K_2HPO_4$, $KH_2PO_4$, $Ca(H_2PO_4)_2$, $CaHPO_4$, $Ca_3(PO_4)_2$, $(NH_4)(H_2PO_4)$, and $Na_3PO_4$, wherein, preferably, the chelating agent is $KH_2PO_4$. The inventors have found that the hydrolysate with reduced salt content is particularly advantageous for a subsequent use as a growth medium if the chelating agent is selected from $Na_3PO_4$, $Na_2HPO_4$, $NaH_2PO_4$, $K_3PO_4$, $K_2HPO_4$, $KH_2PO_4$, $Ca(H_2PO_4)_2$, $CaHPO_4$, $Ca_3(PO_4)_2$, $(NH_4)(H_2PO_4)$, and $Na_3PO_4$. Particularly, the inventors have found that, when such chelating agents are used for the production of a hydrolysate with reduced salt content, the resulting hydrolysate with reduced salt content allows for a particularly efficient production of target products with microorganisms grown on such a hydrolysate as a growth substrate. In one embodiment, said adding a chelating agent to the hydrolysate of step c) comprises adding the chelating agent in a concentration in a range of from 0.001 g/l to 500 g/l, preferably in a range of from 1 g/l to 150 g/l.

In one embodiment, the method comprises adjusting a pH of the hydrolysate of step c) and/or adjusting a pH of the hydrolysate of step d) to a pH in a range of from about pH 2 to about pH 10. In one embodiment, the method comprises adjusting a pH of the hydrolysate of step c) and/or adjusting a pH of the hydrolysate of step d) to a pH in a range of from about pH 3 to about pH 8.5, such as in a range of from about pH 5 to about pH 8.5 or in a range of from about pH 5 to about pH 8; preferably in a range of from about pH 3.5 to about pH 7.5; more preferably in a range of from about pH 5 to about pH 7; even more preferably in a range of from about pH 6 to about pH 7.

In one embodiment, adjusting the pH comprises adding an acid or a base. In one embodiment, the method comprises step e) of adjusting a pH of the hydrolysate, and the adjusting is performed by adding NaOH, KOH, $CH_3COOH$, HCl, KCl, sulfuric acid, phosphoric acid, acetic acid, hydrocyanic acid, carbonic acid, or any combination thereof, preferably by adding NaOH and/or KOH, to the hydrolysate of step c) and/or step d). The inventors have found that the hydrolysate is particularly advantageous for a subsequent cultivation of microorganisms, if the adjusting is performed by adding NaOH, KOH, $CH_3COOH$, HCl, or any combination thereof. In one embodiment, the adjusting is performed by adding NaOH, KOH, $CH_3COOH$, HCl, or any combination thereof.

In one embodiment, e.g. when the hydrolysate with reduced salt content is to be used for a cultivation of a microorganism, adjusting the pH comprises adjusting the pH to a pH of from about pH 3 to about pH 8.5, such as of from about pH 5 to about pH 8.5 or of from about pH 5 to about pH 8. In one embodiment, e.g. when the hydrolysate with reduced salt content is to be used for a cultivation of an acidophilic microorganism and/or of an alkaliphile microorganism, said adjusting the pH comprises adjusting the pH to a pH of from about pH 2 to about pH 10. In one embodiment, e.g. when the hydrolysate with reduced salt content is to be used for a cultivation of an oleaginous microorganism such as a *Cutaneotrichosporon sp.,* said adjusting the pH comprises adjusting the pH to a pH of from about pH 6 to about pH 7, e.g. of about pH 6.5. Advantageously, by adjusting the pH of the hydrolysate, the hydrolysate has a pH which is suitable for the cultivation of microorganism. For example, a pH of about 6.5 may be provided for the use of the hydrolysate for a highly efficient fermentation of microorganisms such as oleaginous yeasts. Particularly, the pH of the hydrolysate may be adjusted such that it matches the need of a microorganism of interest, such as a microorganism to be cultivated in a method of producing a target product of the invention.

In one embodiment, the method comprises a sterilization, preferably of the hydrolysate with reduced salt content obtained in step f), to obtain a sterile hydrolysate with reduced salt content. In one embodiment, the method comprises a sterilization of the hydrolysate which comprises salt provided in step a), of the neutralized hydrolysate obtained in step b), of the hydrolysate which comprises precipitated salt obtained in step c), of a mixture obtained in step d), of a mixture obtained in step e), and/or of the hydrolysate with reduced salt content obtained in step f). In one embodiment, the method comprises one or more steps of sterilization. In one embodiment, the sterilization comprises a thermal sterilization, an ultra-high temperature processing, and/or a sterile filtration. In one embodiment, a step of sterilization comprises a thermal sterilization, an ultra-high temperature processing, and/or a sterile filtration. Advantageously, by sterilizing the hydrolysate, the hydrolysate becomes suitable as a growth medium for cultivation of microorganisms. Particularly, by sterilizing the hydrolysate, contaminations of the growth medium for the cultivation of microorganisms can be prevented. In one embodiment, said hydrolysate with reduced salt content obtained in step f) is filtered through active carbon. The inventors have found that the content of toxic phenols and furans is efficiently reduced by filtering the hydrolysate through active carbon.

In one embodiment, said obtaining a hydrolysate with reduced salt content comprises obtaining a hydrolysate in which the salt content is reduced by at least 1%, preferably by at least 10%, more preferably by at least 30%, even more preferably by at least 50%, even more preferably by at least 80%, compared to the hydrolysate which comprises salt provided in step a). In one embodiment, said hydrolysate with reduced salt content obtained in step f) is sterilized. In one embodiment, said hydrolysate with reduced salt content obtained in step f) is suitable as a growth medium. In one embodiment, said hydrolysate with reduced salt content obtained in step f) is for use in a method of producing a target product of the invention. In one embodiment, said hydrolysate with reduced salt content obtained in step f) comprises sugar such as xylose, glucose, mannose, and/or galactose, and/or comprises an organic acid such as acetic acid. In one embodiment, the hydrolysate with reduced salt content obtained in step f) comprises carbon in an amount in a range of from about 0.1% to about 65% by weight; wherein, preferably, "by weight" relates to a dry weight of the hydrolysate. In one embodiment, said hydrolysate with reduced salt content obtained in step f) is to be used in a method of producing a target product of the invention. In one embodiment, said hydrolysate with reduced salt content obtained in step f) is provided in the form of a growth medium. In one embodiment, said hydrolysate with reduced salt content obtained in step f) is a hydrolysate intended to be used for a microbial cultivation, e.g. for a method of producing a target product of the invention.

The present invention also relates to a method of producing a target product, preferably a microbial oil, comprising the steps:

i) providing a hydrolysate with reduced salt content by performing a method of reducing a salt content of a hydrolysate which comprises salt, as defined herein;
ii) cultivating a microorganism, preferably an oleaginous microorganism, using a growth medium comprising or consisting of the hydrolysate provided in step i), thereby allowing the microorganism to produce the target product; preferably the oleaginous microorganism to produce microbial oil;

iii) optionally, enzymatically treating the microorganism, preferably the oleaginous microorganism; wherein, optionally, said enzymatically treating comprises performing an enzymatic treatment of said microorganism without any solvent-based extraction or chemicals-based demulsification;

iv) obtaining the target product, preferably microbial oil.

The term "target product", as used herein, relates to any product of interest, particularly to any product which can be produced by a microorganism, such as microbial oils, glycerol, free fatty acids, mono- di- and triglycerides, phospholipids, sphingolipids, polyols, alcohols such as ethanol, organic acids, biodiesel, hydrogen, methane, biopolymers, carotenoids, cellulose, squalene, sterols, vitamins, phenolic compounds, pigments, peptides, proteins such as enzymes, DNA, RNA, or other products of interest. The target product may be any product which is of interest for the skilled person, such as microbial oil, microbial oil comprising compounds of interest, and/or compounds obtained from microbial oil. For example, microbial oil may comprise compounds such as antioxidants and other lignin-derived compounds.

In one embodiment, the target product is selected from microbial oils, polyols, alcohols such as ethanol, organic acids, biodiesel, biopolymers, carotenoids, cellulose, squalene, sterols, vitamins, phenolic compounds, proteins such as enzymes, DNA, RNA, other lignin-derived compounds, proteins such as enzymes, DNA, RNA, and any combination thereof. In one embodiment, the target product comprises any compound derived from lignin. In one embodiment, said other products of interest and/or said other lignin-derived compounds may be any compound derived from lignin, preferably selected from Pyrocatechuate, 2,3-Dihydroxybenzoic acid, Coniferyl aldehyde, Syringaldehyde, Methyl vanillate, 3,4-Dihydroxybenzaldehyde, Gentisate aldehyde, Umbelliferone, 3 Hydroxycoumarin, Asaronic acid, Sinapate, Genipin, 2,4,5-trimethoxybenzoic acid, Vanillactic acid, 3-(4-hydroxy-3,5-dimethoxyphenyl)prop-2-enoic acid, 2,4,4'-Trimethoxy-3',6-dihydroxybenzophenone, Ferulaldehyde, 3-Formylphenol, 1-Hydroperoxy-4-methoxybenzene, coumarin, vanillin, Methylphthalicanhydride, Ayapanin, Indole, Benzeneacetonitrile, 5-Aminopentanal, Syrinic acid, Ethyl syringate, Pyroglutamylleucine, 4-(2-Methoxyphenyl)furan-2(5H)-one, 2-(4-Hydroxy-1-benzofuran-2-yl)acetic acid, 1-(4-Hydroxy-3-methoxyphenyl)penta-1,4-dien-3-one, 2,3,6,7-tetramethoxynaphthalene, 1,3,6,8-tetramethoxynaphthalene, Dimethyl 2-[(2-methoxyphenyl)methylidene]butanedioate, 5,10-dimethoxy-2,2-dimethylpyrano[3,2-g]chromen-8-one, Swietenocoumarin F, 4-N-[(3,4-dimethoxyphenyl)methylideneamino]-5-nitropyrimidine-4,6-diamine, 3-OH-3'',4'',5,7-tetraMeO Flavone, 1-(2,4,6-Trihydroxyphenyl)-3-(3,4,5-trimethoxyphenyl)propan-1-one, Syringaresinol, Lenampicilina, beta-D-Glucopyranoside, Tubocurarine, Psymberin, Psymberin, Mesaconitine, aschantin, Phenylpropanolamine, Monomethyl phenylphosphonate, Herniarin, 4-Methylumbelliferone, Chromone, 2-Benzofurancarboxaldehyde, Glycocoumarin, Imbricaric acid, Phenethyl rutinoside, Fluticasone 17beta-carboxylic acid, Gentisic acid, Protocatechuic acid, 7-Azaindolizine, Syringaresinol.

In one embodiment, said phenolic compound is selected from the group consisting of tocopherols, e.g. $\alpha$-, $\beta$-, $\gamma$-, and $\delta$-tocopherol; tocotrienols, e.g. $\alpha$-, $\beta$-, $\gamma$-, and $\delta$-tocotrienol; tocomonoenols, e.g. $\alpha$- and $\beta$-tocomonoenol; phytoestrogens; chalcones, e.g. arbutin, phloretin, phloridzin, and chalconaringenin; flavonoids, e.g. flavonols, flavones, flavanones, flavanols, flavan-3-ols, anthocyanidins, isoflavones, and condensed tannins; and non-flavonoids, e.g. coumarins and phenolic acids. In one embodiment, said vitamin is selected from vitamin A, vitamin $B_1$, vitamin $B_2$, vitamin $B_3$, vitamin $B_5$, vitamin $B_6$, vitamin $B_7$, vitamin $B_9$, vitamin $B_{12}$, and vitamin K.

The terms "microbial lipids" and "microbial oil", as used herein, relates to lipids produced by oleaginous microorganisms, e.g. yeast oil, bacterial oil, and/or fungal oil. In one embodiment, the term "microbial lipid" is used interchangeably with "single cell oil" or "microbial oil". Typically, microbial lipids are rich in unsaturated fatty acids. In one embodiment, the microbial lipids are edible microbial lipids. Such microbial lipids can be used to prepare foodstuff comprising the microbial lipids. Advantageously, microbial oil can be used to replace fats with high saturated fatty acid content and/or fats that are environmentally unfriendly such as palm oil.

In one embodiment, said cultivating a microorganism, preferably an oleaginous microorganism, of step ii) comprises using a growth medium comprising or consisting of the hydrolysate provided in step i) as a growth substrate for the microorganism. Particularly, the growth substrate comprises a carbon source, a nitrogen source, and/or a phosphate source. In a preferred embodiment, the hydrolysate provided in step i) comprises a carbon source, a nitrogen source, and/or a phosphate source. For example, the growth medium may comprise the hydrolysate as a growth substrate, particularly to provide carbon, nitrogen, and/or phosphate for said microorganism. In one embodiment, the term "allowing the microorganism to produce the target product", as used herein, relates to providing suitable growth conditions to the microorganism. In one embodiment, the microorganism produces the target product as a coproduct of microorganism growth, such as a coproduct of a fermentation. In one embodiment, said microorganism produces the target product during cultivation.

In one embodiment, the growth medium comprises said hydrolysate, and further comprises other components, such as a buffer, trace elements, vitamins, and/or further carbon, nitrogen, and/or phosphate sources.

In one embodiment, said microorganism is selected from yeasts, fungi, bacteria and microalgae. In one embodiment, said microorganism is an oleaginous microorganism, preferably an oleaginous yeast. In one embodiment, said microorganism is selected from *Rhodosporidium sp., Yarrowia sp., Rhodotorula sp., Candida sp., Lipomyces sp., Cutaneotrichosporon sp., Trichosporon sp.,* preferably selected from *Cutaneotrichosporon sp.*, more preferably *Cutaneotrichosporon oleaginosus* e.g. *Cutaneotrichosporon oleaginosus* (ATCC 20509). In one embodiment, the microorganism is an oleaginous microorganism, preferably an oleaginous yeast, more preferably a *Cutaneotrichosporon sp.,* even more preferably *Cutaneotrichosporon oleaginosus*. In one embodiment, the microorganism is an oleaginous microorganism and the target product comprises or consists of microbial oil. In one embodiment, said target product comprises or consists of microbial oil, wherein said microbial oil optionally comprises squalene, sterols, vitamins, and/or phenolic compounds.

In one embodiment, said microorganism can be a wild type or genetically modified microorganism. In one embodiment, the genetic modification can be in the form of accelerated evolution, directed evolution, random mutagenesis, and/or targeted engineering. In one embodiment, accelerated evolution, directed evolution, and/or random mutagenesis comprise(s) genomic modifications with UV-treatment, chemical treatment, genetic breeding, error-prone PCR, or other PCR-based methods such as gene shuffling, agrobacteria-mediated transformation, selection, and/or screening. In one embodiment, targeted engineering comprises genomic integration, modification, knock-out, gene knock-down with CRISPR-Cas, agrobacteria-mediated transformation, zink-finger nucleases (ZFN), Transcription activator-like effector nucleases (TALEN), targeted mutagenesis, site-directed mutagenesis with promoter modifications, RNAi, siRNA, and/or combinations thereof. In one embodiment, the object of the genetic modification is to improve the microorganism's acceptability to the salt reduced hydrolysate, improve the microorganism's acceptability to salt content in cultivation conditions, improve the targeted product yield, and/or change the target product's chemical and/or physical properties and/or the composition thereof.

In one embodiment, the hydrolysate with reduced salt content provided in step i) comprises acetic acid and/or sugars such as xylose. In one embodiment, the hydrolysate comprises xylose, acetic acid, and optionally glucose. In one embodiment, the hydrolysate with reduced salt content provided in step i) comprises or consists of a lignocellulosic hydrolysate. In one embodiment, the hydrolysate with reduced salt content provided in step i) is the hydrolysate obtained in step f) of a method of reducing a salt content of the invention. In one embodiment, the hydrolysate with reduced salt content provided in step i) is obtained by a method of reducing a salt content of the invention.

In one embodiment, said cultivating a microorganism, preferably an oleaginous microorganism, using a growth medium comprising or consisting of the hydrolysate provided in step i), comprises a fermentative cultivation of said microorganism, wherein said target product is produced as a coproduct of said fermentative cultivation. In one embodiment, said cultivating a microorganism using a growth medium comprising or consisting of the hydrolysate provided in step i) comprises cultivating said microorganism

for a period of from 1 to 7 days, preferably of from 2 to 4 days;
at a temperature in the range of from 10 °C to 45 °C, preferably in the range of from 15 °C to 40 °C, more preferably in the range of from 20 °C to 33 °C;
at a pH in the range of from pH 4 to pH 9.5, preferably in the range of from pH 5 to pH 8.5, more preferably in the range of from pH 5.5 to pH 8;
in a medium selected from minimal nitrogen media, minimal phosphate media, minimal sulfate media, and acetate rich media, wherein said medium comprises said hydrolysate provided in step i); and/or
with dissolved oxygen ($pO_2$) in a range of from 10 % to 90 %, preferably of from 20 % to 80 %, more preferably of from 30 % to 65 %.

Advantageously, by said cultivation, the microorganism produces the target product.

The term "growth medium", as used herein, relates to a cell culture medium, preferably comprising a carbon source, a nitrogen source, and/or a phosphate source. In one embodiment, the terms "medium" and "growth medium" are used interchangeably. In one embodiment, said growth medium comprises or consists of the hydrolysate provided in step i), wherein said hydrolysate provides a carbon source, a nitrogen source, and/or a phosphate source. In one embodiment, said carbon source comprises glucose, xylose, and/or acetic acid. In one embodiment, said nitrogen source comprises ammonium salts, nitrate salts, amino acids, peptides, N-acetylglucosamine, peptone, yeast extract, and/or urea. In one embodiment, said phosphate source comprises any of organic phosphate compounds, e.g. parathion, malathion, phospholipids, ATP, ADP, AMP, and organophosphate compounds; and inorganic phosphate salts, e.g. $H_3PO_4$, $M_2HPO_4$, $MH_2PO_4$, $MHPO_4$, and $MPO_4$, wherein M is a metal ion.

In one embodiment, the growth medium comprises a carbon source and optionally a buffer, salt, trace elements, a nitrogen source, peptone, and/or a yeast extract. For example, the growth medium may comprise sugar in an amount in a range of from about 10 g/l to about 100 g/l, e.g. of about 30 g/l, and/or acetic acid in an amount in a range of from about 10 g/l to about 50 g/l, e.g. of about 30 g/l. For example, the growth medium may comprise any of $Na_2HPO_4$, $KH_2PO_4$, $CH_3COO\cdot Na$, $MgSO_4\cdot 7H_2O$, $CaCl_2\cdot 2H2O$, $ZnSO_4\cdot 7H_2O$, $MnCl_2\cdot 6H_2O$, $CuSO_4\cdot 5H_2O$, $C_6H_8O_7\cdot Fe\cdot H_3N$, urea, peptone, yeast extract, and combinations thereof.

In one embodiment, said growth medium has a weight ratio of carbon to nitrogen (C:N) < 200, more preferably ≤ 100, even more preferably in a range of from 5 to 80. In one embodiment, said growth medium has a weight ratio of carbon to phosphate (C:P) < 500, more preferably ≤ 150, even more preferably in a range of from 25 to 100. The inventors have found that a medium having a weight ratio of carbon to nitrogen (C:N) < 100, more preferably ≤ 80, even more preferably in a range of from 10 to 80, and/or having a weight ratio of carbon to phosphate (C:P) < 500, more preferably ≤ 150, even more preferably in a range of from 10 to 100, allows to achieve a high yield of said target product(s). In one embodiment, said cultivating of step ii) comprises or consists of a nitrogen-limited fermentation, e.g. using a growth medium having a weight ratio of carbon to nitrogen (C:N) in a range of from 50 to 200.

In one embodiment, said cultivating the microorganism comprises subjecting the microorganism to suitable growth conditions. In one embodiment, the terms "cultivating" and "growing" are used interchangeably. In one embodiment, said cultivating a microorganism, preferably an oleaginous microorganism, using a growth medium comprising or consisting of the hydrolysate provided in step i), comprises a fermentative cultivation of said microorganism. In one embodiment, the term "fermentative cultivation", as used herein, relates to growing a microorganism, such as a yeast, under fermentation conditions. For example, fermentation relates to cultivating a microorganism, particularly a heterotrophic microorganism, using a feedstock, such as aerobic and/or anaerobic cultivation. For example, fermentation conditions may comprise fermentation at a temperature in the range of from 10 °C to 45 °C, preferably in the range of from 15 °C to 40 °C, more preferably in the range of from 20 °C to 33 °C; at a pH in the range of from pH 4 to pH 9, preferably in the range of from pH 5 to pH 8, more preferably in the range of from pH 5.5 to pH 7.5; in a medium selected from minimal nitrogen media, minimal phosphate media, minimal sulfate media, and acetate rich media; and/or with dissolved oxygen (pO2) in a range of from 5 % to 90 %, preferably of from 20 % to 80 %, more preferably of from 30 % to 60 %. In one embodiment, step ii) of cultivating a microorganism comprises a fermentative cultivation. In one embodiment, said step ii) of cultivating a microorganism is performed in fed-batch manner, in semi-continuous mode-manner, or in continuous mode-manner, preferably in continuous mode-manner.

In one embodiment, said step ii) of cultivating a microorganism in a growth medium comprises using said hydrolysate, and optionally an additional feed, as a substrate. In one embodiment, said hydrolysate used as a substrate for growing said microorganism comprises a carbon source, a nitrogen source, and/or a phosphate source. In one embodiment, said hydrolysate provided in step i) is used as a substrate for growing said microorganism in step ii).

In one embodiment, said growth medium in step ii) comprises a carbon source, a nitrogen source, a phosphate source, an organic acid, a trace metal, and/or a vitamin. In one embodiment, said growth medium comprises any of monosaccharides, preferably pentoses or hexoses, more preferably glucose, xylose, mannitol, arabinose, fructose, mannose, sorbitol, lactose, sucrose; oligosaccharides; amino acids; fatty acids; organic acids, preferably acetic acid; minerals; vitamins; trace elements; and combinations thereof. In one embodiment, the trace metal is selected from Mo, Cu, Zn, Mn, Ni, and Fe. In one embodiment, the vitamin is selected from vitamin C, vitamin B, vitamin A, and vitamin E. In one embodiment, said nitrogen source is selected from the group consisting of organic nitrogen compounds, e.g. amines, amides, alkyl nitrates, nitrosamines, nitroarenes, and peroxyacyl nitrates; inorganic nitrogen compounds, e.g. ammonium salts, nitrate salts, and nitrite salts; amino acids; peptides; protein hydrolysates, e.g. peptone, tryptone, and other peptidic hydrolysates, wherein peptidic hydrolysates preferably comprise animal tissue, plant tissue, microbial biomass, and/or components of said yeast; N-acetylglucosamine; and urea; preferably selected from the group consisting of ammonium salts, amino acids, peptides, N-acetylglucosamine, and urea. In one embodiment, said phosphate source is selected from the group consisting of organic phosphate compounds, e.g. parathion, malathion, phospholipids, ATP, ADP, AMP, and organophosphate compounds; and inorganic phosphate salts, e.g. $H_3PO_4$, $M_2HPO_4$, $MH_2PO_4$, $MHPO_4$, and $MPO_4$, wherein M is a metal ion.

In one embodiment, said organic acid is selected from acetic acid, malonic acid, oxalic acid, citric acid, propionic acid, valeric acid, acrylic acid, crotonic acid, butyric acid, isobutyric acid, isovaleric acid, 3-hydroxybutyric acid, 3-hydroxypropionic acid, 2-hydroxybutyric acid, lactic acid, the respective salt(s) of such acids, and combinations thereof. Preferably, said organic acid is acetic acid. It should be noted that the term "organic acid", as used herein, is meant to encompass the respective organic acid irrespective of its degree of protonation, i.e. it is meant to encompass said acid in its protonated state(s) as well as in its deprotonated state(s), e.g. when in aqueous solution at a pH at which it is protonated or deprotonated, respectively, depending on the respective pKa-value(s). The term "organic acid", as used herein, is also meant to encompass salt(s) of the organic acid, e.g. the respective metal salts of such organic acid. Examples of such metal salts are the alkali salts or earth alkaline salts of the respective organic acid. The salts may be in their dissociated form or in their undissociated form. Without wishing to be bound by any theory, the present inventors believe that the presence of organic acid, e.g. of acetic acid allows even further increasing the productivity of the method of producing a target product of the invention, whereas the presence of a carbon source allows an increase in the total biomass.

In one embodiment, the cultivating in step ii) comprises adding further amounts of the hydrolysate provided in step i), lignocellulosic hydrolysate such as lignocellulosic hydrolysate other than the hydrolysate provided in step i), a carbon source other than lignocellulosic hydrolysate, and/or additional nutrients to the growth medium;

wherein, preferably,
said further amounts of the hydrolysate provided in step i), said lignocellulosic hydrolysate, said carbon source, and/or said additional nutrients is/are added in the form of a feed comprising or consisting of volatile organic acid, wherein, preferably, the pH of the feed is in a range from pH 3 to pH 7, preferably pH 3.5 to pH 6, more preferably pH 4 to pH 5.5, wherein, preferably, the volatile organic acid is present in said feed in an amount in a range of from 1 mol/l to 20 mol/l, preferably of from 1.75 mol/l to 15.75 mol/l,
wherein, optionally, said feed further comprises a carbon source other than acetic acid.

In one embodiment, the cultivating in step ii) comprises adding one or more carbon sources and/or additional nutrients in the form of a feed;

wherein, optionally, the feed is provided in any of a continuous, a semi-continuous, a consumption-based, a pH-based, a dissolved-oxygen based, off-gas $CO_2$ concentration based, a staggered manner, and/or a combination thereof;
wherein preferably, the feed is provided in a continuous and/or consumption-based manner. In one embodiment, the terms "providing a feed" and "adding a feed", as used herein, relate to adding a feed to the growth medium, particularly during the cultivation, e.g. in a continuous and/or consumption-based manner.

In one embodiment, said growth medium comprises said hydrolysate as a carbon source. In one embodiment, the cultivating in step ii) comprises adding acetic acid and/or a carbon source other than acetic acid, such as sugars, to the growth medium. For example, the growth medium maybe supplemented with said hydrolysate, and optionally with additional acetic acid and/or an additional carbon source other than acetic acid. For example, said acetic acid may be added to said growth medium in the form of a feed comprising or consisting of acetic acid. In one embodiment, the term "feed" relates to a liquid feed, e.g. to an input stream, and/or to a solid feed. In one embodiment, the feed comprises said hydrolysate and optionally acetic acid. For example, the feed, particularly liquid feed, may comprise liquid acetic acid and/or a liquid comprising acetic acid. In one embodiment, said feed comprises a concentration of acetic acid in a range of from 1 mol/l to 20 mol/l, preferably of from 1.75 mol/l to 15.75 mol/l. In one embodiment, said feed comprises said hydrolysate with reduced salt content and optionally comprises a buffer. In one embodiment, said feed comprises said hydrolysate with reduced salt content, optionally in diluted form, and further comprises acetic acid. In one embodiment, said feed further comprises a carbon source other than acetic acid.
In one embodiment, said growth medium is configured, e.g. by adjusting the respective amount of hydrolysate present in the growth medium, to comprise biodegradable carbon in an amount of 10% (w/v) or less, preferably of 7% (w/v) or less, more preferably of 5% (w/v) or less, e.g. of about 3% (w/v). In one embodiment, said growth medium comprises carbon, particularly sugars and organic acids, in an amount of 10% (w/v) or less, preferably of 7% (w/v) or less, more preferably of 5% (w/v) or less, e.g. of about 3% (w/v).
Biodegradable carbon typically relates to carbon which is degradable and/or usable by microorganisms and/or enzymes; for example, biodegradable carbon is carbon which can be metabolized by microorganisms. In one embodiment, biodegradable carbon comprises sugar(s) and/or organic acid(s) such as acetic acid. In one embodiment, said cultivating comprises a consumption-based feeding, such as a consumption-base acetic acid feeding and/or a consumption-base hydrolysate feeding. The advantage of using a hydrolysate as a feedstock is that it is very cost-efficient and environmentally friendly. Compared to a hydrolysate without reduced salt content, the hydrolysate with reduced salt content has the advantage that growth inhibiting salts are removed, and the efficiency of the cultivation is greatly enhanced.
In one embodiment, said feed comprises about 50% (v/v) acetic acid. In one embodiment, said feed comprises an acetic acid solution, preferably an acetic acid solution of 50% (v/v) acetic acid. In one embodiment, said feed comprises a mixture of said acetic acid solution and said hydrolysate with reduced salt content; wherein, optionally, said acetic acid solution and said hydrolysate are mixed in a ratio of from about 1:1 to about 5:1.
In one embodiment, said cultivating in step ii) comprises providing the growth medium comprising or consisting of the hydrolysate, and further comprises providing a feed, preferably a feed stream, e.g. comprising acetic acid and/or hydrolysate. In one embodiment, the terms "feed stream", "feeding medium", and "substrate feed" are used interchangeably. For example, said growth medium may comprise an initial amount of said hydrolysate and optionally acetic acid, and said growth medium may be supplemented during the cultivation with a feed comprising a further amount of said hydrolysate provided in step i). The inventors have found that a feed comprising acetic acid and hydrolysate maximizes hydrolysate consumption and conversion. In one embodiment, said cultivating of step ii) comprises providing a growth medium comprising or consisting of an initial amount of the hydrolysate provided in step i), and optionally further comprises adding further amounts of the hydrolysate provided in step i) and/or adding acetic acid to said growth medium during the cultivation. In one embodiment, said adding further amounts of the hydrolysate and/or said adding acetic acid is performed in a consumption-based manner or in a continuous manner. For example, the amount of a carbon source, such as acetic acid and/or sugar, present in the growth medium may be measured, and if necessary to provide suitable growth conditions for the microorganism, a carbon source, for example further amounts of the hydrolysate and/or acetic acid, may be added. In one embodiment, said cultivating of step ii) comprises measuring a carbon content of said growth medium, preferably by HPLC, for example using an Agilent 1260 Infinity II LC system with Diode Array (DA) and Refractive Index (RI) detectors. For separation, a column Rezex ROA-organic H+ 8% from Phenomenex may be used with a mobile phase of 5 mM $H_2SO_4$. An isocratic flow of 0.5 mL/min may be applied over 60 min with an oven temperature of 70 °C. The detection in the RID may be carried out at 40 °C. In one embodiment, the growth medium comprises a sugar; preferably a sugar selected from xylose, glucose, fructose, arabinose, mannose, galactose, and combinations thereof; wherein, preferably, the growth medium comprises said sugar in an amount in a range of from about 0.1 g/l to about 100 g/l, preferably of < 50 g/l. In one embodiment, the growth medium comprises organic acid, preferably acetic acid, in an amount in a range of from about 0.01 g/l to about 100 g/l, preferably of from about 1 g/l to about 50 g/l, more preferably of from about 5 g/l to about 10 g/l. The inventors have found that the cultivation is highly efficient if the growth medium comprises the respective concentrations of sugar and/or organic acid.

In one embodiment, said cultivating of step ii) comprises a continuous feed and/or a consumption-based feed. In one embodiment, said cultivating of step ii) comprises a continuous feed comprising an amount of said hydrolysate provided in step i) and/or comprises a consumption-based feed comprising an organic acid, particularly acetic acid, and/or comprising a carbon source other than said organic acid.

In one embodiment, said cultivating of step ii) comprises measuring a sugar concentration of said growth medium, preferably by HPLC, for example using an Agilent 1260 Infinity II LC system with Diode Array (DA) and Refractive Index (RI) detectors. For separation, a column Rezex ROA-organic H+ 8% from Phenomenex may be used with a mobile phase of 5 mM $H_2SO_4$. An isocratic flow of 0.5 mL/min may be applied over 60 min with an oven temperature of 70 °C. The detection in the RID may be carried out at 40 °C. In one embodiment, said cultivating of step ii) comprises measuring an organic acid concentration, such as an acetic acid concentration, of said growth medium, preferably by HPLC, for example using an Agilent 1260 Infinity II LC system with Diode Array (DA) and Refractive Index (RI) detectors. For separation, a column Rezex ROA-organic H+ 8% from Phenomenex may be used with a mobile phase of 5 mM $H_2SO_4$. An isocratic flow of 0.5 mL/min may be applied over 60 min with an oven temperature of 70 °C. The detection in the RID is carried out at 40 °C. In one embodiment, if a measured carbon concentration, sugar concentration, and/or acetic acid concentration is too low to provide suitable growth conditions for said microorganism, e.g. a sugar concentration below 0.1 g/l and/or an organic acid concentration below 0.01 g/l, said cultivating comprises adding further amounts of the hydrolysate, adding sugar, and/or adding acetic acid. In one embodiment, said cultivating comprises adding a feed, preferably a feed stream, to said growth medium; wherein said feed, preferably said feed stream, comprises hydrolysate and optionally additional acetic acid. For example, the feed may comprise about 500 ml of acetic acid per l of feed and about 500 ml hydrolysate per l of feed. In one embodiment, the feed comprises about 10% (v/v) acetic acid to about 100% (v/v) acetic acid, preferably about 10% (v/v) acetic acid to about 90% (v/v) acetic acid. In one embodiment, the feed comprises about 10% (v/v) to about 100% (v/v) of said hydrolysate, preferably lignocellulosic hydrolysate. In one embodiment, said feed comprises acetic acid and said hydrolysate at a ratio of from 10:1 to 1:10. In one embodiment, a carbon content, a sugar content, and/or an organic acid content is measured using HPLC.

In preferred embodiments, the hydrolysate comprises organic acid such as acetic acid. In one embodiment, said growth medium comprises hydrolysate and optionally additional organic acid, such as acetic acid. For example, if the microorganisms need high amounts of organic acids, additional organic acids may be added to said growth medium, particularly in addition to the organic acids comprised by said hydrolysate. For example, a feed and/or a growth medium may comprise organic acid comprised by said hydrolysate and may additionally comprise further organic acid. In one embodiment, the growth medium and/or the feed comprises the hydrolysate and is additionally supplemented with organic acid and/or a carbon source. In one embodiment, the term "hydrolysate", as used herein in the context of a method of producing a target product, relates to a hydrolysate with reduced salt content, particularly to a hydrolysate with reduced salt content provided in step i) of the method of producing a target product according to the invention and/or to a hydrolysate with reduced salt content obtained in step f) of a method of reducing a salt content of a hydrolysate according to the invention.

In one embodiment, said method of producing a target product comprises a step of lysing said microorganism, particularly after step ii) of cultivating the microorganism. For example, by lysing the microorganism, obtaining the target product may be facilitated. In one embodiment, said step of lysing said microorganism comprises any of enzymatic hydrolysis, temperature shock, chemical treatment, high-pressure homogenization, ultrasound homogenization, and any combination thereof.

In one embodiment, the method comprises a step of enzymatically treating the microorganism by performing an enzymatic treatment of said microorganism without any solvent-based extraction or chemicals-based demulsification, preferably by performing a purely enzymatic treatment of said microorganism without any solvent-based extraction or chemicals-based demulsification. In one embodiment, the term "a purely enzymatic treatment of said microorganism without any solvent-based extraction or chemicals-based demulsification" is meant to refer to an enzymatic treatment of said microorganism in which there is a) no extraction using one or several solvents or b) no demulsification using one or several (suitable) chemical reagents or c) neither of a) and b). Preferably such term is meant to refer to an enzymatic treatment devoid of any exposure to an extracting solvent and devoid of any exposure to a demulsifying chemical reagent. The term is also meant to exclude the performance of any other pretreatment of said grown microorganism, for example of oleaginous microorganism. It should be noted that in embodiments of the invention, the "purely enzymatic treatment" excludes the performance of any pretreatment of the grown microorganism, which pretreatment may be chemical (using one or several chemical reagents to which the grown microorganism would be exposed) or physical (such as the change of a physical condition, e.g. temperature, pressure, exposure to ultrasound and/or light, irradiation with electromagnetic radiation etc.). In one embodiment, said purely enzymatic treatment of said microorganism is a treatment of said microorganism with a hydrolase, alone, or in combination with/followed by a protease. In one embodiment, said hydrolase has been obtained from a fungus, preferably from a filamentous fungus, more preferably from a fungus from the Genus *Trichoderma, Aspergillus, Penicillium, Aureobasilium and Fusarium.* In one embodiment, said hydrolase is obtained from a fungus that has been cultured in the presence of an inducing system, wherein, preferably, said inducing system is a component of said microorganism cultivated in step ii).

In one embodiment, said obtaining the target product comprises harvesting the target product by density-based separation, drying, floating, solvent-based extraction, chromatography, distillation, maceration, supercritical fluid extraction, enfleurage, press extraction, demulsification, decantation, and/or aspiration, preferably by density-based separation. In one embodiment, said obtaining the target product comprises harvesting said target product from said medium, from a produced microbial oil, e.g. from an unsaponifiable matter of a produced microbial oil, and/or from a cell debris, preferably from an unsaponifiable matter of a produced microbial oil, optionally after a step of oil extraction. In one embodiment, the terms "obtaining" and "harvesting" are used interchangeably. In one embodiment, the target product is obtained and/or harvested using any of centrifugation, filtration, distillation, organophilic pervaporation, solid-phase micro extraction, and combinations thereof. In one embodiment, said method of producing a target product is performed in fed-batch manner, in semi-continuous mode-manner, or in continuous mode-manner, preferably in continuous mode-manner.

In one embodiment, said method of producing a target product further comprises purifying said target product obtained in step iv), preferably using a separation method comprising chromatography, affinity-based separation, organic solvent extraction, ionic-liquid extraction, supercritical fluid extraction, liquid-liquid extraction, solid phase extraction, flash extraction, steam extraction, vacuum distillation, distillation under inactive or noble gases, and/or deodorization.

Hydrolysates, particularly lignocellulosic hydrolysates, are highly advantageous for fermentation of microorganisms such as oleaginous microorganisms, since the hydrolysates typically comprise organic acid such as acetic acid and comprise sugars. The sugars present in the hydrolysate allow for an efficient first growth phase of the oleaginous microorganism, and the organic acid such as acetic acid allows for an efficient oil production phase. The inventors have found that the cultivation is particularly efficient if the hydrolysate comprises xylose, acetic acid, and optionally glucose.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. In one embodiment, the terms "at least one" and "one or more" are used interchangeably. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm 20\%$, by $\pm 15\%$, by $\pm 10\%$, and for example by $\pm 5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It has been shown that a co-feeding of sugar and acetic acid during cultivation results in high lipid yields circumventing the problems of a limited fermentation and reaching a high lipid titer. The inventors have shown that target products can be efficiently produced with a method of the invention, e.g. when using an oleaginous microorganism such as C. *oleaginosus* for the production of SCOs. Advantageously, C. *oleaginous* can use phenolic compounds as carbon source, such as coumarat and resorcinol, which are major components of depolymerized lignin and are often detrimental to microbial growth. C. *oleaginosus* can efficiently utilize monomers from the most abundant biopolymers on earth, cellulose, chitin, and lignin as well as hemicellulose, as the yeast metabolizes a variety of different sugars, including xylose, glucose, and derivate N-acetylglucosamine. In addition to its metabolic flexibility, inhibitory compounds like hydroxymethylfurfural (HMF) have little impact on the lipid productivity of *C. oleaginosus.* The inventors have found that, advantageously, microorganisms such as *C. oleaginosus* can efficiently use biogenic waste streams, particularly hydrolysates, e.g. from the pulp and paper industry, if the salt content of such hydrolysates is reduced prior to the use of the hydrolysate for the cultivation of the microorganisms.

Advantageously, the method of reducing a salt content of a hydrolysate which comprises salt allows converting hydrolysates, such as lignocellulosic hydrolysates, which comprise salts in amounts that inhibit microbial growth, to hydrolysates with reduced salt content which can be used as growth substrates for microbial growth. For example, such hydrolysates with reduced salt content, e.g. spent liquor with reduced salt content, can be used as a carbon source for a microbial production of target products such as microbial oil. Advantageously, the method of producing a target product of the invention, which uses the hydrolysate as a growth substrate, is much more cost-efficient and environmentally friendly than methods of producing target products using glucose as a growth substrate for microbial growth. Thus, the economic performance is enhanced.

In one embodiment, the method of producing a target product, preferably a microbial oil, comprises the steps:

i) providing a hydrolysate with reduced salt content by performing the steps of

a) providing a hydrolysate which comprises salt, preferably a biogenic hydrolysate which comprises salt, more preferably a lignocellulosic hydrolysate which comprises salt;

b) optionally, neutralizing a pH of the hydrolysate of step a) to obtain a neutralized hydrolysate; wherein, optionally, the neutralized hydrolysate has a pH in a range of from about pH 4 to about pH 8;

c) adding $CaCO_3$, $Ca(OH)_2$, $CaO$, $MgO$, $MgCO_3$, and/or $Mg(OH)_2$, preferably $CaCO_3$ and/or $Ca(OH)_2$, to the hydrolysate of step a) or to the neutralized hydrolysate of step b) to obtain a hydrolysate which comprises precipitated salt; wherein, optionally, the hydrolysate which comprises precipitated salt has a pH in a range of from about pH 4 to about pH 8.5;

d) adding a chelating agent to the hydrolysate of step c);

e) optionally, adjusting a pH of the hydrolysate of step c) and/or step d) to a pH in a range of from about pH 3 to about pH 8.5, preferably of from about pH 3.5 to about pH 7.5, more preferably of from about pH 5 to about pH 7, even more preferably of from about pH 6 to about pH 7; and

f) obtaining a hydrolysate with reduced salt content;

ii) cultivating a microorganism, preferably an oleaginous microorganism, using a growth medium comprising or consisting of the hydrolysate provided in step i), particularly the hydrolysate obtained in step f); thereby allowing the microorganism to produce the target product; preferably the oleaginous microorganism to produce microbial oil;

iii) optionally, enzymatically treating the microorganism, preferably the oleaginous microorganism; wherein, optionally, said enzymatically treating comprises performing an enzymatic treatment of said microorganism without any solvent-based extraction or chemicals-based demulsification;

iv) obtaining the target product, preferably microbial oil.

Advantageously, the hydrolysate provided in step i) results in a growth characteristic similar or even more efficient compared to the growth characteristics using only xylose, glucose and/or acetic acid as a carbon source, and is much more cost-efficient.

BRIEF DESCRIPTION OF THE FIGURES

The present invention is now further described by reference to the following figures.

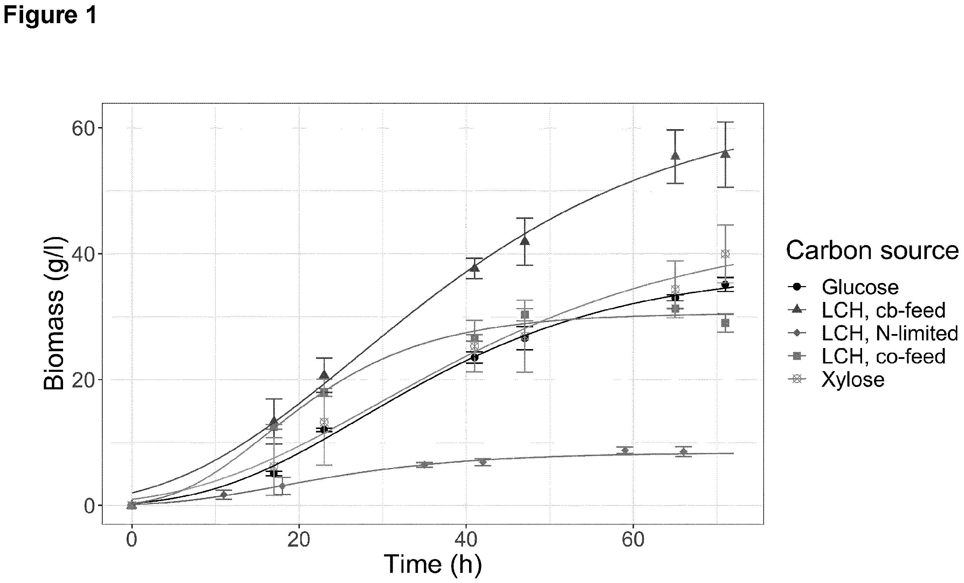

**Figure 1** shows growth behavior of different fermentation conditions in comparison (LCH - Lignocellulosic hydrolysate, N-limited - nitrogen limited fermentation conditions, cb-feed - consumption-based feeding, co-feed - combination of consumption-based feed and continuous feed of LCH). Biomass accumulation after inoculation of fermentations with different starting sugars and acetic acid consumption-based feeding in all cases but the nitrogen limited conditions, curve fitting with Gompertz function. Error bars display two-times standard deviation.

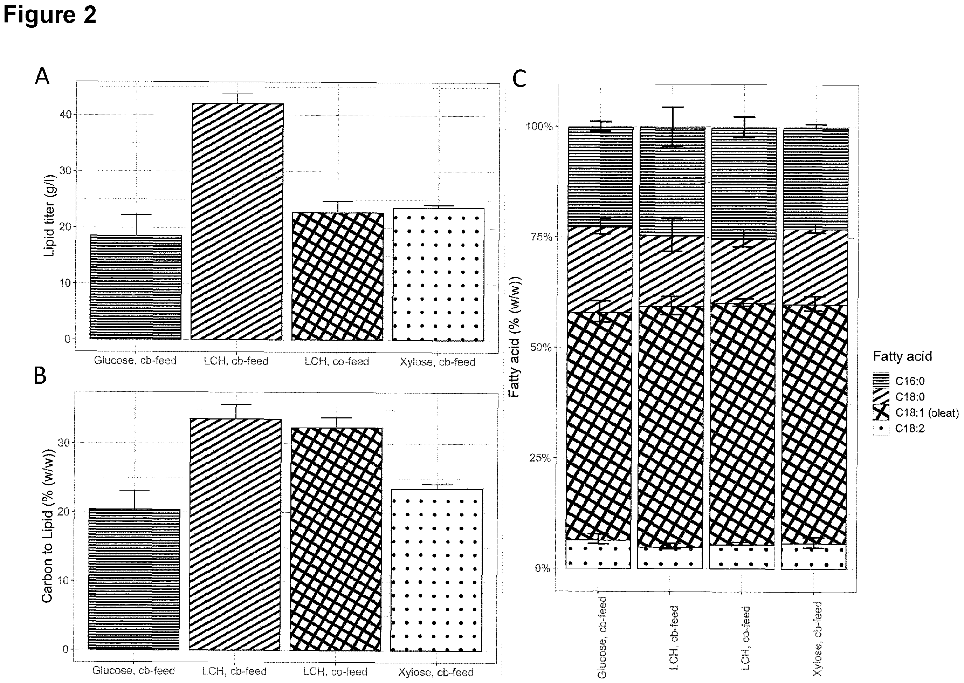

**Figure 2** shows lipid analysis of the five most important fermentation conditions. Starting carbon sources are abbreviated with: Glu - Glucose, LCH - Lignocellulosic hydrolysate, LCH co-feed - Lignocellulosic hydrolysate as starting carbon and constant feed, Xyl - Xylose. (a) Lipid titers after 71 h fermentation in 1 L scale. (b) Carbon conversion from substrate carbon to lipid carbon. (c) fatty acid profile of the main fatty acids quantified with GC-FID. Error bars display two-times standard deviation.

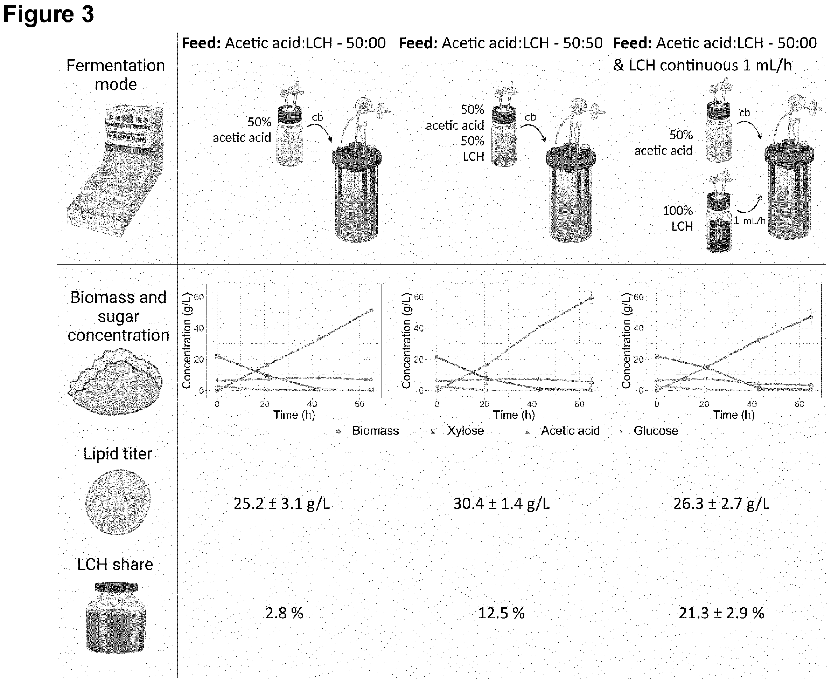

**Figure 3** shows a comparison of the feeding strategies at 0.25 L scale in the DASbox® system (LCH - Lignocellulosic hydrolysate). Biomass accumulation and substrate consumption for the control (50% acetic acid, consumption-based feed) and the best two operation modes (co-feeding with acetic acid:LCH at 50:50 and continuous feeding with LCH at 1 ml/h). Total lipid titers achieved after 65 h and share of LCH on the total carbon uptake. Error bars display two-times standard deviation.

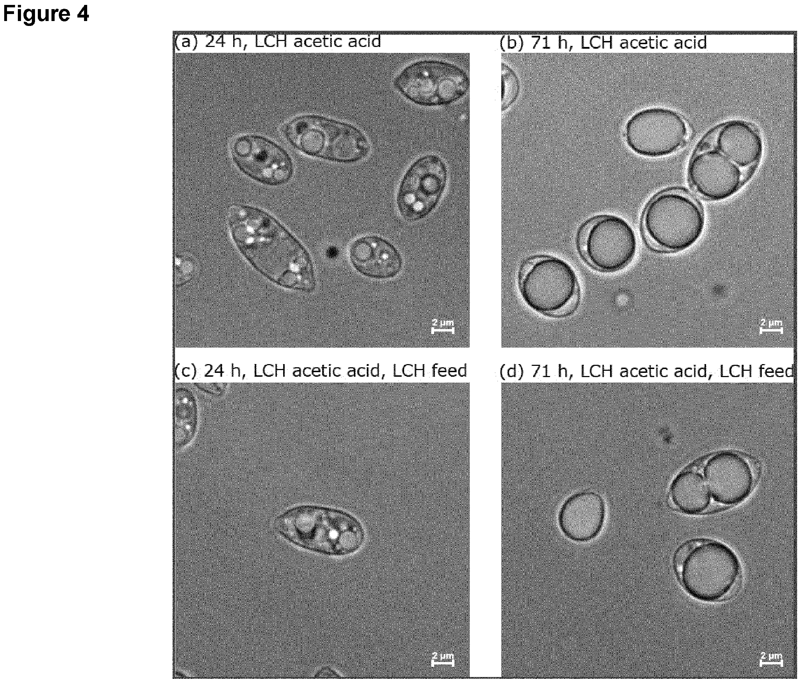

**Figure 4** shows confocal microscopy images of lignocellulosic hydrolysate fermentation with acetic acid-based feeding after 24 h (a) and after 71 h (b) as well as cells from LCH continuous feeding combined with acetic acid feeding after 24 h (c) and after 71 h (d).

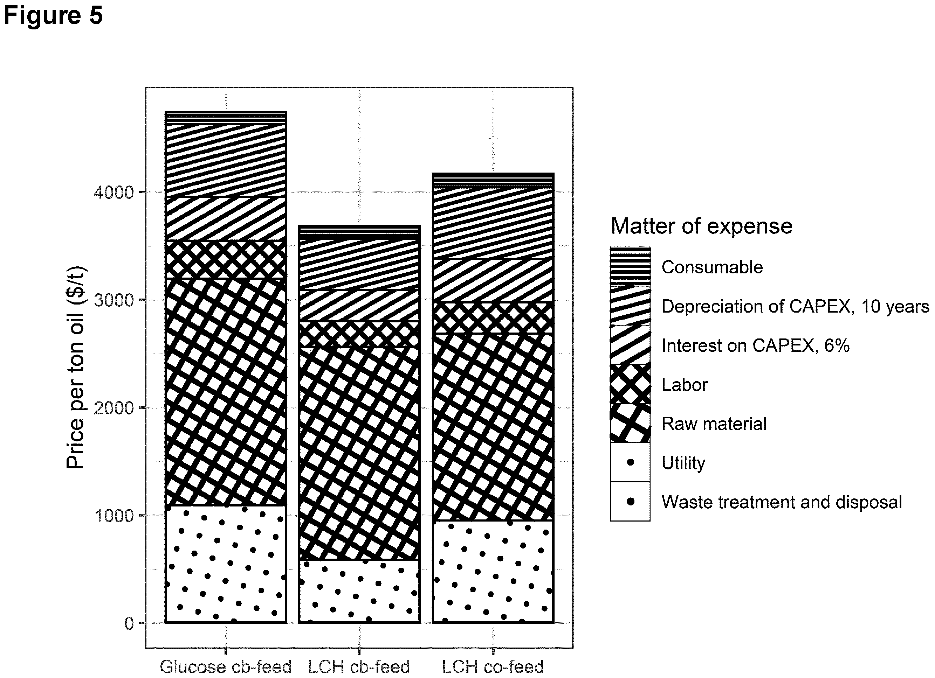

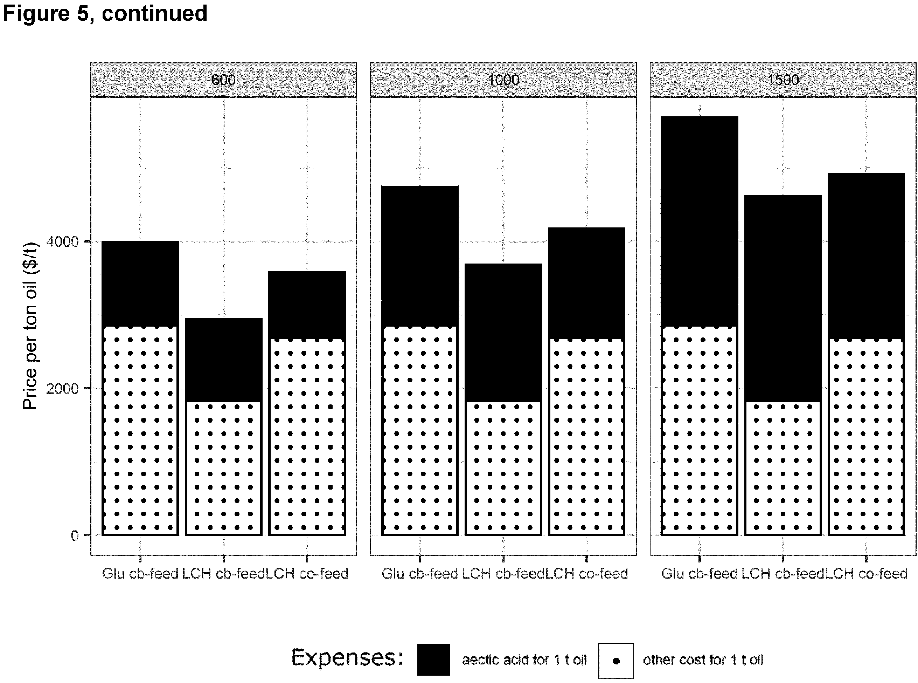

**Figure 5** shows annual production cost of yeast oil in $/Mt for the three fermentation strategies analyzed with the TEA. LCH cb-feed - LCH with consumption-based feeding of acetic acid, Glucose cb-feed - Glucose with consumption-based feeding of acetic acid, LCH co-feed - continuous feeding of LCH and consumption-based feeding of acetic acid. The respective amount of feedstock was set to produce yeast oil at a rate of 0.81 Mt/h (LCH cb-feed: 1 Mt/h, Glucose cb-feed: 0.151 Mt/h, LCH co-feed: 2.1 Mt/h).

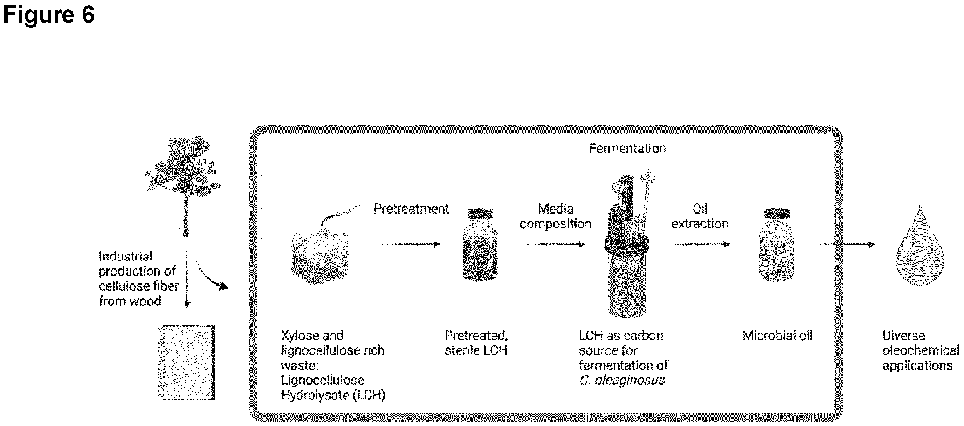

**Figure 6** shows an exemplary embodiment of the method of the invention.

All methods mentioned in the figure descriptions below were carried out as described in detail in the examples. In the following, reference is made to the examples, which are given to illustrate, not to limit the present invention.

EXAMPLES

**Example 1:** Material and Methods

Sugar analysis

Sugars and short organic acids were analyzed with High-performance liquid chromatography (HPLC). All samples were filtered with a 10 kDa filter. The Agilent 1260 Infinity II LC system with Diode Array (DA) and Refractive Index (RI) detectors was used. For separation a column Rezex ROA-organic H+ 8% from Phenomenex was used with a mobile phase of 5 mM $H_2SO_4$. An isocratic flow of 0.5 ml/min was applied over 60 min with an oven temperature of 70 °C. The detection in the RID was carried out at 40 °C.

Element analysis

Elemental analysis was carried out with a Euro EA CHNS elemental analyzer (HEKAtech Ltd.). Dynamic spontaneous combustion in a Sn boat at approximately 1800°C was performed with subsequent gas chromatographic separation and was detected using a thermal conductivity detector (TCD).

Ash

Neutralized and lyophilized lignocellulose hydrolysate of 2 g to 4 g was burned at 1000°C for 3 h to ash. The amount was determined gravimetrically after cooling overnight in a desiccator.

Dry weight

The dry weight of substrate solutions and biomass samples were determined gravimetrically. To measure the dry cell weight 4 mL of fermentation culture were transferred to pre-weighed tubes, centrifuged (4500 rcf, 20 min) and washed two times with equal amount of water or 50% EtOH in case of lipid-rich cells. Alternatively, 0.5 ml of a lipid-rich culture were filtered through a pre-weighed 0.2 μm filter paper and washed tree times with 2 ml water. The samples were frozen and lyophilized. For each biological replicate at least technical duplicates were measured.

Quantification of sulfates

The quantification of sulfates in the hydrolysate was done chemically with treatment with $CaCO_3$ and $BaCl_2$. The resulting $BaSO_4$ precipitate was quantified gravimetrically.

Lipid content

For lipid content analysis the cells from fermentation were centrifuged and washed two times with 50% EtOH and resolved in water. The cells were disrupted mechanically with a High Pressure Homogenizer Type HPL6 from Maximator. Triplicates of 7 ml disrupted cell solution were frozen and lyophilized. The chloroform methanol lipid extraction was carried out after modified Bligh and Dyer. Shortly, 100-200 mg biomass were weight in a glass tube, 4 ml $Cl_3CH$:MeOH (2:1) and 1 ml $H_2O$ (0.58% NaOH) were added. After 60 min shaking at 120 rpm, it was centrifuged for 10 min at 2000 rcf and the bottom layer was transferred to a new glass tube. Additional 3 ml $Cl_3CH$:MeOH (2:1) were added to the upper phase in the first tube and shortly mixed. After centrifugation the bottom layer was transferred to the tube containing the firth extract and 2 ml of $H_2O$ (0.58% NaOH):MeOH (1:1) were added. After mixing and centrifugation the bottom phase was transferred to a fresh pre-weight glass tube and the solvents were evaporated under nitrogen flow. The lipid amount was determined gravimetrically.

### Fatty acid profile

The fatty acid profile was measured through gas chromatography after fatty acid methyl esterification (FAME) of unwashed and lyophilized samples from the fermentation process. A known amount between 3 to 10 mg was weighed in glass vials, all further steps were automated with the Multi Purpose Sampler MPS robotic from Gerstel. An internal standard of 10 g/l C19 TAG in toluol was used for quantification. First, 490 $\mu$l toluol and 10 $\mu$l internal standard were added and mixed for 1 min at 1000 rpm, afterwards 1 ml 0.5 M sodium methoxide in methanol were added and the solution was heated to 80°C and shaked at 750 rpm for 20 min. After cooling to 5°C 1 ml 5% HCl in methanol was added and the mixture was heated to 80°C while shaking at 800 rpm for 20 min and cooled to 5°C afterwards. 400 $\mu$l dd$H_2O$ was added and mixed for 30 s at 1000 rpm, before 1 ml hexane was added and it was extracted by shaking tree times for 20 s at 2000 rpm in quickMix device. The samples were centrifuged for 3 min at 1000 rpm and cooled at 5°C before a 200 $\mu$l sample of the organic phase was transferred to a 1.5 ml-vial for chromatography. The gas chromatograph flame ionization detection (GC-FID) was used to quantify the fatty acids. GC-MS was carried out to identify the acids with the TRACE™ Ultra Gas Chromatograph from Thermo Scientific coupled to a Thermo DSQ™ II mass spectrometer and a Triplus™ Autosampler injector in positive ion mode. A Stabilwax® fused silica capillary column (30 m × 0.25 mm, film thickness 0.25 $\mu$m) was used for separation. The temperature profile for the analysis was set to an initial column temperature 50°C, increasing at a rate of 4°C/min up to a final temperature of 250°C. Hydrogen was used as carrier gas at a constant flow rate of 35 ml/min. Standardization was done with the FAMEs Marine Oil Standard (20 components from C14:0 until C24:1)

### Pretreatment LCH

For precipitation of sulfates within the hydrolysate, 20 g of $CaCO_3$ was added to 1 l of LCH in and 5 l beaker while constantly stirring. The hydrolysate was then until complete outgassing of $CO_2$. Next, the LCH was frozen at -20°C over night. After defrosting, the LCH was centrifuged at 10000 rpm for 10 minutes and the supernatant was collected. 20 g of $KH_2PO_4$ was added to the remaining liquid for removal of excess calcium and the pH was adjusted with 3 M NaOH to 7. The LCH was centrifuged at 10000 rpm for 10 minutes and sterile filtered to be used in the cultivation experiments.

### OD measurement for process monitoring

Cell growth was monitored by measuring the optical density at 600 nm ($OD_{600}$) of culture samples. Samples were diluted in the respective cultivation media to an $OD_{600}$ between 0.1 and 1.

### Strains and preculture

50 mL YPD media (10 g/l yeast extract, 20 g/l peptone, and 20 g/l glucose) in an Erlenmeyer flask containing antibiotics (0.05 g/l kanamycin, 0.1 g/l ampicillin) was inoculated with a single colony of C. *oleaginosus* (ATCC 20509) from a YPD plate. Flasks were incubated at 28 °C under constant shaking at 120 rpm for 2 days. These yeast precultures were used as inoculum in the different fermentation setups.

### Fermentation Media

Different media were used for either nitrogen limitation or co-fermentation with acetic acid. The base media was composed of 0.9 g/l $Na_2HPO_4$, 2.4 g/l $KH_2PO_4$, 2 g/l $MgSO_4 \cdot 7H_2O$, 0.5 $CaCl_2 \cdot 2H_2O$, 0.00000055 g/l $ZnSO_4 \cdot 7H_2O$, 0.000024 g/l $MnCl_2 \cdot 6H_2O$, 0.000025 g/l.

Techno-economic analysis

In order to estimate the total capital investment and operation cost for the production of oil from LCH using C. *oleaginosus* techno-economic analysis (TEA) was performed. The process was designed in a consequential approach in silico according to previous laboratory results, available data from the literature and mathematical functions integrated in SuperPro Designer (SPD) version 10 (Intelligen, Inc., Scotch Plains, NJ, USA). In the process simulation a production plant was created with a lipid production capacity of 0.81 metric tons per hour (t/h). The feedstock requirements and the chemicals needed for LCH pretreatment were estimated based on the media composition used in the current work. The mathematic equations and parameters for simulating yeast biomass generation, lipid formation and enzymatic hydrolysis were based on results generated in this work or in previous experiments. To calculate energy balance, equipment sizing and purchasing prices the values from the SPD database were completed with publicly available current prices and data from the literature. The in silico plant featured several operations for LCH pretreatment, fermentation and downstream processing, including recovery of single cell oil and recycling of waste streams and side products. The list of modules used in SPD for the different fermentation conditions (glucose and LCH with acetic acid co-feeding, combination of continuous and consumption-based feeding) is included in the supplements.

From all process parameters, lipid productivity has the highest impact on cost 1. Lipid productivity was deduced from the the lipid titres from the fermentations performed in the 1 L DASGIP® system after 71 h.

$$\text{Lipid productivity [g/l/h]} = \text{lipid titer [g/l]} \,/\, \text{time [h]}$$

The lipid productivities were calculated for three conditions: glucose with acetic acid co-feeding, LCH with acetic acid co-feeding as well as the combination of continuous feed and consumption-based acetic acid co-feeding. Equipment purchase cost (PC) was calculated with an internal SPD function based on size of the equipment. Installation cost factors relative to the PC were included with values between 1.1 and 1.3. Cost factors for storage piping and site development were set as 4%, 9% and 5%.

Capital interest was included as 6% of the total investment. Utility and labor cost as well as waste disposal were estimated based on the current prices in Germany. Cost for consumables and raw materials were set according to current market prices. Maintenance and insurance were included as faculty-dependent costs as 3% and 0.7%.

**Example 2**: Results and Discussion

Analysis of lignocellulosic hydrolysate

The lignocellulosic hydrolysate (LCH), from spent liquor of the pulping process, was comprehensively analyzed to gain a better understanding of its composition, in order to set up a pretreatment strategy. The dark brown solution contains a small share of insoluble particles. It had a pH of 1.7 and a dry mass of 247.7 $\pm$ 13.9 g/l. A high sulfate content of 19.4 $\pm$ 2.0 g/l was determined. HPLC analysis was used to quantify sugar, organic acids and furans. It showed that the primary sugar of the hydrolysate was xylose with 77.05 $\pm$ 2.46 g/l and smaller amounts of other sugars like glucose (11.51 $\pm$ 0.39 g/l), mannose (8.21 $\pm$ 0.84 g/l), galactose (6.47 $\pm$ 0.21 g/l) and others (12.37 $\pm$ 1.10 g/l), resulting in a total sugar content of 115.60 $\pm$ 4.99 g/l. Organic acids had a total concentration of 16.83 $\pm$ 1.43 g/l, acetic acid being the major one with 12.34 $\pm$ 1.20 g/l. A considerable amount of 4.53 $\pm$ 0.59 g/l hydroxymethylfurfural (HMF) and 0.68 $\pm$ 0.04 g/l furfural was detected. The total ash was 0.70 $\pm$ 0.01 g/l, with low concentrations of phosphorus (0.035 g/l) and no detectable nitrogen. The rest of the material derives from lignols and lignans well as other plant metabolites, being a total amount of 89.95 $\pm$ 22.97 g/l. The hydrolysate is a waste stream from an industrial production of cellulose fibers using hardwood as source material and an acidic pulping process. The chemical hydrolysis of lignocellulose results in the detected sugar monomers, organic acids, lignols and lignans. The containing phenolic compounds and furans might be problematic for the fermentation process, as they generally have an inhibitory effect on microbial growth. The high sulfate content and the acidic pH result from the chemical hydrolysis. Therefore, the LCH needs to be neutralized for its application in yeast fermentation. The high content of xylose and other sugars though make it an ideal carbon source. Due to the very low amounts of nitrogen, phosphorus, and other elements like sulfur, magnesium, or calcium, additional nutrient supplementation is required before using the hydrolysate in a fermentation medium.

Pretreatment of the lignocellulosic hydrolysate

To transform the waste stream LCH into a suitable carbon source for yeast fermentation, the challenges of low pH and lack of essential nutrients needed to be addressed with a suitable pretreatment strategy. In the first step, LCH was neutralized with different methodologies and sterilized. Afterwards it was mixed with essential salts, buffer compounds, nitrogen sources, nutrients, and trace elements. The resulting media were tested in small scale for the cultivation of C. *oleaginosus* in 24 deep-well plates. Neutralization with NaOH from pH 1.7 to pH 7 led to a high concentration of salts resulting in reduced growth. To reduce the amount of sulfate, $CaCO_3$ was added reacting to partially insoluble $CaSO_4$. The addition of 20 g/l $CaCO_3$ proved to be a slight excess but could increase the pH only to 5.6. Therefore, NaOH was used for the titration to neutral, resulting in a solution with lower salt content and better growth behavior. For sterilization different thermal methods were tested, but all of them resulted in the formation of insoluble particles. Therefore, filtration was used for sterilization. Nevertheless, insoluble particles were formed again after the addition of the other medium compounds like buffering salts, vitamins, nitrogen, and trace elements, as well as during the cultivation. In a different approach, the LCH was filtered through active carbon to reduce the content of toxic phenols and furans. Unfortunately, this strategy also reduced the amount of available carbon source and therefore reduced the growth drastically.

Over the fermentation time the formation of insoluble particles was noted, contributing to calcium phosphate due to high $[Ca^{+2}]$. Therefore, LCH was titrated with $KH_2PO_4$ to remove the excessive calcium before the fermentation in the form of insoluble $Ca_3(PO_4)_2$. As this process results in acidification, the final neutralization with NaOH was done after the $KH_2PO_4$ treatment. This pretreated LCH resulted in a growth similar to the one with the control with xylose, glucose and acetic acid (XGA), details to the pretreatment experiments are provided in the supplementary data.

In this manner, the lignocellulosic hydrolysate was neutralized, without high salt concentrations and without formation of insoluble material when buffering salts or trace elements were added. The sugar content was maintained thus it could be applied as carbon source for C. *oleaginosus.* This optimized pretreatment strategy was applied to all LCH used for fermentation in this study.

## Nitrogen-limited fermentation

Fermentation under nutrient limitation, like phosphate or nitrogen stress, is a common strategy to trigger lipid accumulation in oleaginous yeast. Therefore, nitrogen-limited fermentation was performed using LCH as carbon source. To understand the effect of the lignocellulose derived compound on the yeast metabolism, a control medium was conceived. The control contained only the main sugars from the LCH in their respective ratio: xylose (8.28%), glucose (1.03%), and acetic acid (0.69%) and is therefore abbreviated with XGA. The fermentation was carried out as fed-batch on a scale of 1 l maximal volume in the DASGIP® system. In the 500 ml starting media the sugar content of 3% from either LCH or XGA was used. The nitrogen content was adjusted in order to accomplish a C/N ratio of 120. Pretreated pure LCH or XGA was continuously fed, starting after 12 h with a rate of 10 ml/h and 5 ml/h between 36 and 60 h. The fermentation resulted in a biomass formation of 7.02 $\pm$ 0.88 g/l and 16.65 $\pm$ 0.24 g/l for LCH and XGA, respectively, showing better growth performance on the XGA medium. Visual observation under the microscope revealed the formation of several lipid droplets in each cell.

Presumably, the high concentration of inhibitory furans and other phenolic compounds could explain the inhibited growth on lignocellulosic hydrolysate compared to growth on XGA. Generally, nitrogen limitation induces lipid formation, but restricts biomass formation at the same time, as nitrogen is required for protein synthesis and other metabolic processes.

## Acetic acid-based fermentation on sugars as carbon source

As discussed earlier, it has been shown that an increased growth as well as increased lipid accumulation of C. *oleaginosus* can be achieved by combining sugar-containing starting medium with consumption-based acetic acid feeding. Glucose, xylose as well as the mixed control of xylose, glucose, and acetic acid (XGA) as starting sugars were tested with consumption-based acetic acid feeding. The concentration of acetic acid was constant due to the consumption-based feeding; the sugars were consumed by the first 40 h after inoculation. The biomass increased further after the complete consumption of the sugar and reached 35.11 $\pm$ 1.11 g/l, 39.95 $\pm$ 4.59 g/l and 39.00 $\pm$ 0.76 g/l for glucose, xylose, and XGA by 71 h, with lipid titers of 18.5 $\pm$ 3.6 g/l, 23.6 $\pm$ 0.5 g/l and 21.6 $\pm$ 2.8 g/l for glucose, xylose, and XGA, respectively (Fig. 2a). The same applied to the carbon conversion from substrate carbon to lipid carbon that is 20.4 $\pm$ 2.7%, 23.0 $\pm$ 1.4% and 23.5 $\pm$ 0.7% for glucose, xylose, and XGA (Fig. 2b). The fatty acid profile of the oil is shown in figure 2c, the most abundant fatty acid is C18:1 with around 54% followed by C16:0 (~ 24%), 18:0 (~ 15%), and C18:2 (~ 6%) and traces of C18:3, C16:1 and C22:0 with less than 0.3% each.

The fermentation of glucose and xylose as sole carbon sources showed a similar growth behavior like the fermentation using XGA. This indicates a good and simultaneous uptake of both glucose and xylose. This is a considerable advantage for C. *oleaginosus* as fermentation strain because the full potential of the biomass as carbon sources can be realized. Moreover, the acetic acid can be efficiently channeled into the metabolism of lipid synthesis by Acetyl-CoA synthetase. Resulting in a more efficient metabolic pathway then starting from sugars molecules and is therefore an effective way to increase the lipid biosynthesis of C. *oleaginosus.*

Acetic acid-based fermentation of lignocellulosic hydrolysate

The successful production of single cell oil from refined sugars with the acetic acid-based fermentation was shown. For a commercially more attractive process though the mentioned waste stream, LCH, was applied as carbon source in place of the costly sugars. Lignocellulose hydrolysate is the spent liquor from acidic hardwood pulping, beneficially this process also produces acetic acid by the cleavage of acetyl groups from the hardwood xylose. The second carbon base for the process, acetic acid, can therefore be produced on site.

A natural product such as lignocellulosic hydrolysate contains furans and phenols that might have an inhibitory effect on the organism. To determine the optimal balance between carbon supply and toxic level of inhibitory compounds, fermentation with three different starting concentrations of LCH were performed. The amount of hydrolysate was used for a concentration of 3%, 5% and 7% of bioavailable carbon in the starting media. The fermentation with the highest LCH concentration, corresponding to a sugar amount of 7%, showed a longer lag-phase and a slower overall growth with a growth rate of 1.21g/l/h compared with 1.33 g/l/h for 3%. The dry biomass formation was 39.64 $\pm$ 6.30 g/l, 51.92 $\pm$ 0.18 g/l for 7% and 5% next to 58.90 $\pm$ 1.05 g/l in case of 3%. Accordingly, an increase of the concentration to 5% resulted in a slightly longer lag-phase and a slightly lower biomass after three days of fermentation. The lipid titer was similar for the lower two concentrations (25.59 $\pm$ 1.79 g/l and 25.75 $\pm$ 2.02 g/l) but lower for 7% LCH (19.83 $\pm$ 1.38 g/l).

The starting carbon source concentration of 3% resulted in the shortest lag-phase, best growth rate, and overall biomass formation. Whereby, higher concentrations resulted in higher concentrations of inhibitory furans and phenols, explaining the longer lag phase and decreased growth rates. Therefore, the starting concentration of 3% was used for all further experiments.

Acetic acid-based fermentation on lignocellulosic hydrolysate with optimized conditions

The optimized conditions for the fermentation of C. *oleaginosus* on LCH were evaluated by the fermentation of three biological replicates. The starting carbon content of the hydrolysate corresponding to 3% sugar and acetic acid was used. A high biomass formation of 55.73 $\pm$ 5.20 g/l was achieved after 71 h, which is a seven-fold increase compared to the nitrogen-limited fermentation of LCH. Moreover, biomass formation was 50% higher with LCH as carbon source compared to glucose or xylose under equivalent reaction conditions (Fig. 1). However, sugar consumption showed a similar profile as the fermentation processes using sole sugars. The lipid titer after 71 h was twice as high (42.1 $\pm$ 1.7 g/l) as from glucose fermentation (18.5 $\pm$ 3.6 g/l) (Fig. 2a). The carbon conversion was 33.6% carbon lipid from substrate carbon (Fig. 2b) with 76.05 $\pm$ 9.04 % lipid per dry biomass. The fatty acid profile did not show significant changes compared to the lipid profile of the fermentation on single sugars or XGA (Fig. 2c). The optimized fermentation strategy of C. *oleaginosus* on LCH with acetic acid consumption-based feeding showed the best performance in biomass accumulation, lipid titer, and lipid yield with minor changes in the lipid profile. The increased performance on LCH as a substrate might be contributed to the contained lignol and lignan compounds that can partly be metabolized by C. *oleaginosus.* In summary, this combination of waste stream and oleaginous yeast is a promising one for a sustainable new process of single cell oil production. It combines the application of a waste stream rich in xylose, lignols and lignans with a fermentation strategy resulting in high lipid yields.

Screening of different strategies for feeding of LCH

Consumption-based acetic acid feeding results in high lipid productivity of over 85%. Nevertheless, compared to acetic acid, LCH it is a cheap feedstock as it is a waste product with limited options of value creation. In order to increase LCH uptake by the oleaginous yeasts relative to the consumption of acetic acid, different fermentation modes were compared. In this context, the DASbox® twelve parallel bioreactor system was used to simultaneously compare five fermentation conditions in duplicates. All conditions utilized consumption-based feeding with 50% (v/v) acetic acid.

As first fermentation mode combined -feeding of a mix of LCH and acetic was tested. LCH was added to 50% (v/v) acetic acid to a final concentration of either 10% (v/v) and 50% (v/v). The biomass of the cells fed with the 50:10 and 50:50 mix of acetic acid:LCH (51.5 $\pm$ 3.7 g/l and 59.6 $\pm$ 1.2 g/l) exceeded those solely fed with acetic acid after 65 hours (50.8 $\pm$ 0.1 g/l), with 50:50 co-feeding being the highest (Fig. 3). The lipid titers after 65 h were 28.4 $\pm$ 0.4 g/l and 30.4 $\pm$ 1.4 g/l for the 50:10 and 50:50 feeding, respectively, while 25.2 $\pm$ 3.1 g/l were measured for the control settings (Fig 3).

As an additional feeding strategy, the pure pretreated LCH was continuously fed into the reactor throughout the fermentation process at two feeding rates: 0.5 ml/h or 1 ml/h starting from 12 h after inoculation in addition to consumption-based feeding of 50% (v/v) acetic acid. The values for the dry biomass reached after 65 h were 50.4 $\pm$ 2.2 g/l and 50.4 $\pm$ 4.6 g/l for 0.5 ml/h and 1 ml/h, respectively. The maximum lipid titers were 30.5 $\pm$ 2.4 g/l for 0.5 ml/h, and 26.3 $\pm$ 2.7 g/l for 1 ml/h, therefore above the control (25.2 $\pm$ 3.1 g/l).

In order to select the setup with the highest LCH turnover, the share of LCH in the overall consumed carbon was calculated. The strategy using 1 mL/h continuous feed resulted in the highest percentage of LCH consumed (21.3 ± 2.9 %), followed by 50:50 acetic acid:LCH (12.5%) and continuous feed with 0.5 mL/h (10.4 ± 1.2 %). The differences between the duplicates continuously fed with LCH can be explained with different consumed quantities of acetic acid. As the share of LCH using 50:50 acetic acid:LCH stayed constant over the fermentation, therefore no standard deviation could be calculated.

In addition, the carbon conversion rate of fed carbon to lipid was calculated for all conditions. In comparison, the setups using continuous feed had the highest carbon conversion rates with 19.8 ± 2.0 % for 0.5 mL/h and 18.8 ± 0.2 % for 1 ml/h. The control showed a conversion rate of 16.4 ± 0.2 % and co-feeding with the acetic acid:LCH mix resulted in 16.7 ± 0.1 % for a ratio of 50:10 and 16.7 ± 0.5 % for 50:50. The control and the two best feeding strategies are visualized in figure 3.

For overall evaluation of the feeding strategies, the focus was set on the share of consumed LCH and the lipid titer. In regard to this, the two best feeding strategies were cb-feed with 50:50 acetic acid:LCH as well as a co-feed with a continuous feed of 1 ml/h LCH. By providing more LCH during the fermentation process, these strategies could significantly increase the share of LCH. At the same time the starting concentration of inhibitory compounds was not increased which could prevent an elongated lag-phase as observed for higher LCH concentrations in the DASGIP® system (3.5.).

Co-fermentation with continuous feed of lignocellulosic hydrolysate

Using 1 mL/h continuous feed of LCH in the DASbox® setting led to the highest share of LCH consumed of the overall carbon source and had the second highest carbon conversion rate among all tested conditions. Therefore, this condition was selected for the DASGIP® system. The 1 L scale also allowed a better direct comparison of the fermentation output with the other conditions tested in the DASGIP® system. Consumption-based feed with 90% acetic acid was used in combination with a continuous LCH feed of 3.3 ml/h., the biomass accumulation reached 29.0 ± 5.87 g/l after 71 h with a lipid titer of 22.7 ± 1.95 g/l. HPLC analysis showed, that the carbon substrates were evenly consumed throughout the fermentation, only xylose accumulated slightly from 15.5 ± 1.11 g/l at 65 h to 17.44 ± 1.85 g/l at 71 h. The xylose accumulation is presumably caused by a slightly too high feeding rate, resulting in an excessive supply of sugar and an increased concentration of inhibitory compounds. The share of LCH from the total consumed carbon could be increased from 10.9 ± 31.4 % to 37.6 ± 2.3 %. Thereby, the relative amount of LCH consumed by C. *oleaginous* could be increased, which is a more cost efficient feedstock than acetic acid. At the same time, the substrate carbon converted to lipid carbon remained at a similar level of 32.0 ± 1.5 % compared with 33.5 ± 2.1 % from the fermentation solely using acetic acid consumption-based feeding. The cell phenotype and growth behavior as well as the lipid droplet formation was similar for both fermentation strategies, as detected in the Nile red stained cells shown in figure 4. After 24 h of fermentation the cells already start to build several lipid droplets but show an oval shape. After 71 h of fermentation the cells mainly consist of one or maximal two lipid droplets filling most of the cell and giving the cells a circular morphology. Overall, using the operation mode with the additional continuous feeding, substrate to lipid conversion of C. *oleaginosus* was comparable to consumption-based feeding with acetic acid. For further optimizations of the fermentation mode the feeding ratio between acetic acid and sugar should be adapted to avoid sugar accumulation, as indicated by the xylose concentrations after 71 h.

Techno-economic analysis

Aside the cumulative results in this study, we performed a consequential techno-economic analysis (TEA), to assess the economic feasibility of a commercial-scale production plant using LCH as an industrially relevant feedstock. Two different modes of LCH fermentation from the 1 L scale DASGIP® experiments were implemented and compared to potentially reduce cost and maximize LCH utilization: acetic acid consumption-based feeding using LCH as starting carbon source (LCH cb-feed) and the combination of consumption and continuous feeding of LCH (LCH co-feed). Both fermentation modes were compared to an operation set-up, where glucose was used as starting carbon source for consumption-based feeding using acetic acid (Glucose cb-feed). According to the simulation, CAPEX was lowest for LCH cb-feed with 24.4 M $. In comparison, CAPEX in LCH cb-feed was 34.5 M $ and 33.9 M $ for LCH co-feed. The increased capital cost was due to the different amounts and vessel volumes of the fermenters in the respective model. Different fermenter combinations had to be used for the fermentation strategies because of differences in dwell times in the fermentation conditions to simulate the individual lipid productivities. Other than the vessel amounts and volumes the equipment cost was the same. Depreciation was calculated as 10% over ten years resulting in 2.4 M $, 3.4 M $ and 3.4 M $ for LCH cb-feed, Glucose cb-feed and LCH co-feed, respectively. The quantity and volumes of the vessels also resulted in differences in operating costs, especially costs for power (LCH cb-feed: 2.3 M $, Glucose cb-feed: 4.8 M $, LCH co-feed: 4.0 M $), cooling water (LCH cb-feed: 0.5 M $, Glucose cb-feed: 1.0 M $, LCH co-feed: 0.9 M $), labor cost (LCH cb-feed: 1.5 M $, Glucose cb-feed: 2.2 M $, LCH co-feed: 1.8 M $), maintenance (LCH cb-feed: 0.6 M $, Glucose cb-feed: 0.8 $, LCH co-feed: 0.8 M $) and raw materials (LCH cb-feed: 12.4 M $, Glucose cb-feed: 13.2 M $, LCH co-feed: 10.9 $). The final costs for yeast oil calculated from the in silico model were 3,500 $/t, 4,500 $/t and 4,000 $/t, respectfully (fig. 5a). All calculated prices regimes are within the current price range for organic palm oil (2,500-3,000 $/t). Forward-looking, the price points for the LCH processes could potentially be reduced by further optimization of the fermentation processes and resulting lipid conversion rates. Nevertheless, consequential TEA, as applied in this study, focusses more on relative changes of input and output conditions to each other. The calculated prices per t should therefore be only considered to be a range. Other plant oils used for biofuel production, such as canola oil are priced well below 1,000 $/t. However, utilizing an industrial waste stream, circumvents competition with edible oils, thus enhancing the sustainability aspect of the LCH-based oil production processes presented in this study.

In addition, sensitivity analysis was performed considering different acetic acid prices, as it made up the majority of the raw material cost (LCH cb-feed: 94%, Glucose cb-feed: 90%, LCH co-feed: 86%). Acetic acid prices in Western Europe were fluctuating between 600 $/t and 1,500 $/t between 2012 and 2021, so the cost of yeast oil per t is largely dependent on the current acetic acid price. For the sensitivity analysis acetic acid costs of 600 $/t and 1,500 $/t were assumed and compared to 1,000 $/t, the value which was used in the TEA. The mode using LCH cb-feed responded strongest to the price changes, as shown in figure 5b. The price reduction to 600 $/t led to a 19.3% decrease in production costs (3,100 $/t) and an elevated acetic acid price of 1,500 $/t resulted in a 24.1% increase (4,800 $/t). For LCH co-feed the total production costs decreased 13.5% (3,800 $/t) and increased 16.9% (5,100 $/t) for the respective acetic acid prices. The LCH co-feed model reacted less sensitively to changes in acetic prices due to a larger share of LCH in the carbon available to the yeast. Overall though, LCH cb-feed still showed the cheapest production costs for a decrease or increase of the acetic acid prices even though the percental change in price was the highest. The main reason for the fluctuations in acetic acid prices is that the USA and China supply 17% and 55% of the global acetic acid, respectively. Problems or even disruptions in these supply chains have a profound impact on the pricing as the availability might be drastically decreased. Local or even on-site production and supply of acetic acid would be best, to avoid such dependencies.

In summary, our lab-scale experiments for the biotechnological valorization of an industrial waste stream using C. *oleaginosus* were validated in an in silico fermentation setup. Compared to glucose, cost-efficacy of LCH was demonstrated for the production of single cell oil in a biorefinery scale. Such resource-efficient approaches could lead towards more independent, circular and sustainable economies in the future.

Conclusion

Herein provided is a novel strategy for the production of microbial lipids, e.g. with the oleaginous yeast C. *oleaginosus*, using lignocellulosic hydrolysate and acetic acid as main carbon sources. To this end, fermentations were performed in 0.25 l and 1 l scale and a maximal lipid titer of 42.1 ± 1.7 g/l was reached with optimized conditions. LCH consumption was increased to a maximum of 37.6 ± 2.3 % of total consumed carbon. Furthermore, the economic advantages of the use of LCH over glucose were shown in a techno-economic analysis. Concluding, we present a sustainable and economic strategy for the production of single cell oils as an alternative platform for the production of biofuels and oleochemicals.

The features of the present invention disclosed in the specification, the claims, and/or in the accompanying figures may, both separately and in any combination thereof, be material for realizing the invention in various forms thereof.

**Claims**

1. A method of reducing a salt content of a hydrolysate which comprises salt, preferably a biogenic hydrolysate which comprises salt, comprising:

   a) providing a hydrolysate which comprises salt, preferably a biogenic hydrolysate which comprises salt, more preferably a lignocellulosic hydrolysate which comprises salt;
   b) optionally, neutralizing a pH of the hydrolysate of step a) to obtain a neutralized hydrolysate; wherein, optionally, the neutralized hydrolysate has a pH in a range of from about pH 4 to about pH 8;
   c) adding $CaCO_3$, $Ca(OH)_2$, $CaO$, $MgO$, $MgCO_3$, and/or $Mg(OH)_2$, preferably $CaCO_3$ and/or $Ca(OH)_2$, to the hydrolysate of step a) or to the neutralized hydrolysate of step b) to obtain a hydrolysate which comprises precipitated salt; wherein, optionally, the hydrolysate which comprises precipitated salt has a pH in a range of from about pH 4 to about pH 8.5;
   d) adding a chelating agent to the hydrolysate of step c);
   e) optionally, adjusting a pH of the hydrolysate of step c) and/or step d) to a pH in a range of from about pH 3 to about pH 8.5, preferably of from about pH 3.5 to about pH 7.5, more preferably of from about pH 5 to about pH 7, even more preferably of from about pH 6 to about pH 7;
f) obtaining a hydrolysate with reduced salt content.

2. The method according to claim 1, wherein the hydrolysate provided in step a) is a hydrolysate derived from a paper production such as a hydrolysate derived from a pulp production, a hydrolysate derived from forestry, an agricultural hydrolysate, a food hydrolysate, a food waste hydrolysate, a biofuel waste hydrolysate, a textile hydrolysate, an animal tissue hydrolysate, a plant tissue hydrolysate, a microbial biomass hydrolysate, an industrial waste hydrolysate, a municipal waste hydrolysate, or any combination thereof;
wherein, preferably, the hydrolysate is a lignocellulosic hydrolysate, preferably a spent liquor hydrolysate, and/or is a hydrolysate derived from pulping, more preferably is a hydrolysate derived from acidic pulping.

3. The method according to claim 1 or 2, wherein the hydrolysate provided in step a) is a hydrolysate obtained by a physical treatment, a chemical treatment, an enzymatical treatment, and/or a biological treatment of a substrate, preferably of a biomass;

   wherein, preferably,
   the physical treatment is selected from mechanical treatments, pressure treatments, heat treatments, steam explosions, combustion, and any combination thereof;
   the chemical treatment is selected from an alkaline treatment, an acidic treatment, and a treatment at neutral pH; wherein, preferably, the chemical treatment is a treatment with any of a salt, an acid, a peroxide, and any combination thereof, preferably with a sulfide, a sulfite, and/or a bisulfite;
   the enzymatical treatment is a treatment with one or more enzymes selected from hydrolases, preferably endo- and exo-glycoside hydrolases, glycosylases, peptidases, such as endo- and exo-peptidases, proteases, amylases, dehydrogenases, peroxidases, ligninolytic enzymes, and any combination thereof; and
   the biological treatment is a treatment with a microorganism, preferably a treatment with a microorganism selected from bacteria, yeast, and fungi.

4. The method according to any one of the foregoing claims, wherein the hydrolysate provided in step a) comprises salt in an amount in a range of from about 0.0001 mol/l to about 15 mol/l, preferably of from about 0.0005 mol/l to about 8 mol/l, and/or carbon in an amount in a range of from about 0.1% by weight to about 65% by weight.

5. The method according to any one of the foregoing claims, wherein the hydrolysate provided in step a) comprises a salt selected from a sulfate, a sulfide, a sulfite, a nitrate, a nitrite, a chloride, and any combination thereof; wherein, preferably, the salt comprises a sulfate, a sulfide, and/or a sulfite.

6. The method according to any one of the foregoing claims, wherein the hydrolysate provided in step a) comprises a lignol, a lignan, an organic acid, and/or a sugar; wherein, optionally, said sugar comprises xylose, glucose, mannose, and/or galactose; wherein, preferably, said sugar comprises monosaccharides, preferably xylose; and wherein, preferably, said organic acid comprises acetic acid.

7. The method according to any one of the foregoing claims, wherein the chelating agent is selected from $M_3PO_4$, $M_2HPO_4$, $MH_2PO_4$, $MHPO_4$, $MPO_4$, $(NH_4)(H_2PO_4)$, and any combination thereof, wherein M is a metal;

   wherein, preferably, the chelating agent is selected from $Na_3PO_4$, $Na_2HPO_4$, $NaH_2PO_4$, $K_3PO_4$, $K_2HPO_4$, $KH_2PO_4$, $Ca(H_2PO_4)_2$, $CaHPO_4$, $Ca_3(PO_4)_2$, $(NH_4)(H_2PO_4)$, and $Na_3PO_4$;
   wherein, more preferably, the chelating agent is $KH_2PO_4$.

8. The method according to any one of the foregoing claims, wherein the method comprises step e) of adjusting a pH of the hydrolysate, and wherein the adjusting is performed by adding NaOH, KOH, $CH_3COOH$, HCl, KCl, sulfuric acid, phosphoric acid, acetic acid, hydrocyanic acid, carbonic acid, or any combination thereof, preferably by adding NaOH and/or KOH, to the hydrolysate of step c) and/or step d).

9. The method according to any one of the foregoing claims, wherein the method further comprises a sterilization, preferably of the hydrolysate with reduced salt content obtained in step f), to obtain a sterile hydrolysate with reduced salt content;
wherein, preferably, the sterilization comprises a thermal sterilization, an ultra-high temperature processing, and/or a sterile filtration.

**10.** A method of producing a target product, preferably a microbial oil, comprising the steps:

i) providing a hydrolysate with reduced salt content by performing a method according to any one of the foregoing claims;

ii) cultivating a microorganism, preferably an oleaginous microorganism, using a growth medium comprising or consisting of the hydrolysate provided in step i), thereby allowing the microorganism to produce the target product; preferably the oleaginous microorganism to produce microbial oil;

iii) optionally, enzymatically treating the microorganism, preferably the oleaginous microorganism; wherein, optionally, said enzymatically treating comprises performing an enzymatic treatment of said microorganism without any solvent-based extraction or chemicals-based demulsification;

iv) obtaining the target product, preferably microbial oil.

**11.** The method according to claim 10, wherein the microorganism is an oleaginous microorganism, preferably an oleaginous yeast, more preferably a *Cutaneotrichosporon sp.*, even more preferably *Cutaneotrichosporon oleaginosus*.

**12.** The method according to claim 10 or 11, wherein the target product is selected from microbial oils, glycerol, free fatty acids, mono- di- and triglycerides, phospholipids, sphingolipids, polyols, alcohols, organic acids, biodiesel, hydrogen, methane, biopolymers, carotenoids, cellulose, squalene, sterols, vitamins, phenolic compounds, pigments, peptides, proteins such as enzymes, DNA, RNA, and any combination thereof;

wherein, preferably, the target product comprises microbial oil.

**13.** The method according to any one of claims 10-12, wherein the hydrolysate with reduced salt content provided in step i) comprises acetic acid and/or xylose, wherein, preferably, the hydrolysate comprises or consists of a lignocellulosic hydrolysate.

**14.** The method according to any one of claims 10-13, wherein the cultivating in step ii) comprises adding acetic acid and/or a carbon source other than acetic acid to the growth medium;

wherein, preferably,

said acetic acid is added in the form of a feed comprising or consisting of acetic acid, wherein, preferably, a concentration of acetic acid in said feed is in a range of from 1 mol/l to 20 mol/l, preferably of from 1.75 mol/l to 15.75 mol/l,

wherein, optionally, said feed further comprises a carbon source other than acetic acid.

**15.** The method according to any one of claims 10-14, wherein the growth medium comprises a sugar such as xylose in an amount in a range of from about 0.1 g/l to about 250 g/l, preferably < 100 g/l; and/or

wherein the growth medium comprises acetic acid in an amount in a range of from about 0.01 g/l to about 100 g/l, preferably of from about 1 g/l to about 50 g/l, more preferably of from about 5 g/l to about 10 g/l.

**Figure 1**

# Figure 2

EP 4 389 905 A1

# Figure 3

**Figure 4**

(a) 24 h, LCH acetic acid

(b) 71 h, LCH acetic acid

(c) 24 h, LCH acetic acid, LCH feed

(d) 71 h, LCH acetic acid, LCH feed

Figure 5

EP 4 389 905 A1

Figure 5, continued

**Figure 6**

Europäisches
Patentamt

European
Patent Office

Office européen
des brevets

# EUROPEAN SEARCH REPORT

Application Number

EP 22 21 5578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/134836 A2 (OURO FINO PARTICIPACOES E EMPREENDIMENTOS; NETO, D.C.) 13 November 2008 (2008-11-13) * page 3, line 26 - page 4, line 1 * * page 4, lines 12-17 * * page 5, line 25 - page 6, line 4 * * page 9, line 5 - page 11, line 5 * * page 16, line 9 - page 21, line 5; table 4 * * page 35; table 12 * * pages 43-46; claims 1, 7-9, 12 * * figures 1-3 * | 1-15 | INV. C12P7/6463 C13K1/02 C13K13/00 |
| X | DE 30 25 098 A1 (AMERICAN CAN CO.) 8 January 1981 (1981-01-08) * pages 1-9; claims 1-4, 7, 8, 14, 17-20, 24 * * page 15, line 26 - page 18, line 4 * * page 28, line 18 - page 30, line 29 * * page 37, line 1 - page 38, line 2 * | 1-15 | |
| X | WO 2017/205705 A1 (API INTELLECTUAL PROPERTY HOLDINGS, LLC) 30 November 2017 (2017-11-30) * pages 49-52; example 4 * | 1-15 | TECHNICAL FIELDS SEARCHED (IPC) C12P C13K |

The present search report has been drawn up for all claims

| Place of search | Date of completion of the search | Examiner |
|---|---|---|
| Berlin | 6 July 2023 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 5578

06-07-2023

| Patent document cited in search report | | Publication date | Patent family member(s) | | Publication date |
|---|---|---|---|---|---|
| WO 2008134836 | A2 | 13-11-2008 | AU | 2008247252 A1 | 13-11-2008 |
| | | | CR | 10904 A | 06-10-2009 |
| | | | EC | SP099497 A | 29-09-2009 |
| | | | NI | 200900137 A | 29-06-2010 |
| | | | NZ | 578234 A | 21-12-2012 |
| | | | US | 2010330615 A1 | 30-12-2010 |
| | | | WO | 2008134836 A2 | 13-11-2008 |
| DE 3025098 | A1 | 08-01-1981 | BR | 8003397 A | 31-03-1981 |
| | | | DE | 3025098 A1 | 08-01-1981 |
| | | | FR | 2460331 A1 | 23-01-1981 |
| | | | ZA | 801703 B | 27-05-1981 |
| WO 2017205705 | A1 | 30-11-2017 | US | 2017342444 A1 | 30-11-2017 |
| | | | WO | 2017205705 A1 | 30-11-2017 |